# EUROPEAN PATENT APPLICATION

(11) **EP 2 578 696 A1**
(43) Date of publication of application: **10.04.2013**
(21) Application number: 11184241.5
(22) Date of filing: 06.10.2011
(51) Int. Cl.: C12Q 1/68

(54) **MiRNAs as non-invasive biomarkers for diagnosis**

(71) Applicant: febit holding GmbH, 69120 Heidelberg (DE)
(72) Inventor: Keller, Andreas, 66346 Püttlingen (DE); Beier, Markus, 69469 Weinheim (DE); Backes, Christina, D-66125 Saarbrücken-Dudweiler (DE)

(57) **Abstract**

The present invention relates to non-invasive methods, kits and means for diagnosing and/or prognosing of a disease in a body fluid sample from a subj ect. Further, the present invention relates to set of polynucleotides or sets of primer pairs for detecting sets of miRNAs for diagnosing and/or prognosing of a disease in a body fluid sample from a subject.

SEQUENCE LISTING

<110>  febit holding GmbH

<120>  miRNAs as non-invasive biomarkers for diagnosis

<130>  FP-011

<160>  254

<170>  PatentIn version 3.5

<210>  1
<211>  19
<212>  RNA
<213>  Homo sapiens

<400>  1
cgagcccacc cagggacgc                                                    19


<210>  2
<211>  19
<212>  RNA
<213>  Homo sapiens

<400>  2
aagugggagg cccccggcg                                                    19


<210>  3
<211>  19
<212>  RNA
<213>  Homo sapiens

<400>  3
aaggcaaacc uaauaguuu                                                    19


<210>  4
<211>  22
<212>  RNA
<213>  Homo sapiens

<400>  4
aauaccgggu gcuguaggcu ua                                                22


<210>  5
<211>  19
<212>  RNA
<213>  Homo sapiens

<400>  5
gcugagggcc acacccagg                                                    19


<210>  6
<211>  21
<212>  RNA
<213>  Homo sapiens

<400>  6
ccugcacugg aucacuccgu g                                                 21


<210>  7
<211>  28
<212>  RNA
<213>  Homo sapiens

<400>  7
guaagugaag auaaagugug ucugagga                                          28


<210>  8
<211>  19
<212>  RNA
<213>  Homo sapiens

<400>  8
gcccgcccca gccgagguu                                                    19


<210>  9
<211>  19
<212>  RNA
<213>  Homo sapiens

<400>  9
gggccgugga gguggacug                                                    19


<210>  10
<211>  110
<212>  RNA
<213>  Homo sapiens

<400>  10
ucaggaggcu gaggcaggag aaucgcauug aaccugagag gcagagguug caguggcacg       60

aucucagguc acugcaaccu cugccuccgg uauucaagcg auucuccugc                 110


<210>  11
<211>  107
<212>  RNA
<213>  Homo sapiens

<400>  11
gcccgaacga gagguuccgg agccccggcg cgggcggguu cuggggugua gacgcugcug       60

gccagcccgc cccagccgag guucucggca ccgccuugag agcuuca                    107


<210>  12
<211>  110
<212>  RNA
<213>  Homo sapiens

<400>  12
ugugugcaga agguucccug gggcuggugu cacagugcac aacugcaggg gugugaaugu       60

agcaggacgg ggccguggag guggacugug augcuggcuc agcacaauuu                 110


<210>  13
<211>  98
<212>  RNA
<213>  Homo sapiens

<400>  13
gcaggggcuc ugcucuccac auuggagggu guggaagaca ucugggccaa cucugaucuc       60

uucaucuacc ccccaggacu gggacaagcc cgagcaua                               98


<210>  14
<211>  103
<212>  RNA
<213>  Homo sapiens

<400>  14
ucaagagcuu agaaacuguu gacguugcca ugucuaagaa gaaaauuuuu cuccaaaguu      60

uucuucuuag acauggcagc uucagcaguu ucuuccaaag aaa                        103


<210>  15
<211>  19
<212>  RNA
<213>  Homo sapiens

<400>  15
cucuucaucu accccccag                                                    19


<210>  16
<211>  110
<212>  RNA
<213>  Homo sapiens

<400>  16
ccaccuucug aagccuacuu cugucaguuu gucaaacucu uucgccaucc aguuuuguuc      60

ccuugcuggu gaggaguugu gauccuuugg agaagaggca uucuguuuuu                 110


<210>  17
<211>  20
<212>  RNA
<213>  Homo sapiens

<400>  17
gucaguuugu caaacucuuu                                                   20


<210>  18
<211>  94
<212>  RNA
<213>  Homo sapiens

<400>  18
cacaggggcc cuauuuugug caccgcaacc cacacacggu cucagcugcu gagaccgugu      60

guggguugcu gagucacuga agacggcggc ucgg                                  94


<210>  19
<211>  110
<212>  RNA
<213>  Homo sapiens

<400>  19
uuauuuggaa gagggcuuag gugcacgcuc uagcggggau uccaauauug ggccaauucc      60

cccaauguug gaauccucgc uagagcgugc acuucuggaa gcuaggaacc                 110


<210>  20
<211>  110
<212>  RNA
<213>  Homo sapiens

<400>  20
cgggagcgga cugcugucca ggugggugug ggcagugggc gggccaagga cagucccggg      60

gagcugccgg ggggcaguug gcaccguccc cugcgccuac ccacucaccc                 110


<210>  21
<211>  110
<212>  RNA

<213>  Homo sapiens

<400>  21
gcagccuggg acggguagau ucagggugga gcagggcggc uauugugggg gucagccugg      60

gaaggcgucc acaaaccugc cagcccugcc cuccugacga ggggacagcc      110


<210>  22
<211>  104
<212>  RNA
<213>  Homo sapiens

<400>  22
aucccagaga augacucaga aaccgguuga gaugcaaggg cugcugcagg gcuacuagag      60

cugcucuuac aucucaaacg auguccugca ugucaacaaa gccc      104


<210>  23
<211>  32
<212>  RNA
<213>  Homo sapiens

<400>  23
gcaguuggca ccguccccug cgccuaccca cu      32


<210>  24
<211>  110
<212>  RNA
<213>  Homo sapiens

<400>  24
aauguugaaa aacacaacuc cugaaucacc accaaaccug uucuuccucc agggucuccc      60

aagagaacag guuugguggg gauucaggag uuguauuuug uucauguuaa      110


<210>  25
<211>  20
<212>  RNA
<213>  Homo sapiens

<400>  25
ccacaaaccu gccagcccug      20


<210>  26
<211>  110
<212>  RNA
<213>  Homo sapiens

<400>  26
ugacccuugg cccccgauag aaccgagugg cuccuugcac cuguggcugc cuguggaugg      60

ggaaggugcc cagaggauca cggagccacu cgguucuauu gggggucagg      110


<210>  27
<211>  110
<212>  RNA
<213>  Homo sapiens

<400>  27
ccaaacgcuc uucugcguuc cgcugugggg agaggggcag agcacugugu guggaguugg      60

ugggaccuga uuucagcgcu cugccccucu cugucuggcu guaugagugu      110


<210>  28

<211>  21
<212>  RNA
<213>  Homo sapiens

<400>  28
uuggaauccu cgcuagagcg u                                                21


<210>  29
<211>  107
<212>  RNA
<213>  Homo sapiens

<400>  29
gcugugguga cacaccuaaa gagcuggaag ggcaaaagac ugcuacagca ggauguucag      60

ucuucagucu uuugcccuuu cagcucuuuc aguguuuaag gaccuuc                   107


<210>  30
<211>  110
<212>  RNA
<213>  Homo sapiens

<400>  30
cucuuauccg cacuuccaag ccuuggccac cacaccuacc ccuugugaau gucgggcaau      60

gggugauggg uguggugucc acaggcaaca ggggagaagg aggagaugga                110


<210>  31
<211>  21
<212>  RNA
<213>  Homo sapiens

<400>  31
cguugccaug ucuaagaaga a                                                21


<210>  32
<211>  110
<212>  RNA
<213>  Homo sapiens

<400>  32
uucugagaca aaacaacccc ucuccucucc ucccugugcc gaccccacug gcuagaagac      60

gugggaagcg cggggaggga ggauaagggc ucugaaugcu ucugucccca                110


<210>  33
<211>  101
<212>  RNA
<213>  Homo sapiens

<400>  33
ccucccuagg ggaucccagg uaggggcagc agaggaccug ggccuggaca uggcacuccc      60

ugauccucag cugcccucuc ucccacagcc accacugcca g                         101


<210>  34
<211>  22
<212>  RNA
<213>  Homo sapiens

<400>  34
ccugauccuc agcugcccuc uc                                               22


<210>  35

<211>  92
<212>  RNA
<213>  Homo sapiens

<400>  35
uagugcugcc ucccaccgau gcccccuccg agcaggcacu gcucagucug gugccugugc      60

agagggagcu acuucgaagg cgcuaucagu cc                                    92


<210>  36
<211>  20
<212>  RNA
<213>  Homo sapiens

<400>  36
ccaccuucaa aggcacuccg                                                  20


<210>  37
<211>  110
<212>  RNA
<213>  Homo sapiens

<400>  37
ggcuuggagg agcugacguu uaucuggagg ccucugcugg ugcugguugc uggggcagca      60

aguccuccac cuucaaaggc acuccgcucc uccucccucc ugccuucgcc                110


<210>  38
<211>  101
<212>  RNA
<213>  Homo sapiens

<400>  38
caagcagaug ugggccuaca uuuagagccu auccuguuuu gcaaaacugc agggcaugca      60

aaacaggaua ggcacuaaau gguaaaaagu auacuuauuu a                         101


<210>  39
<211>  110
<212>  RNA
<213>  Homo sapiens

<400>  39
accgcggagg acaggggcag cuggcgggca gcgggugagg ggguggcggg gacgcgagug      60

gcggccgcgg ggccccggac aaggguccgc agagcugcag ccuucgaggg                110


<210>  40
<211>  110
<212>  RNA
<213>  Homo sapiens

<400>  40
cucuccauau caccauuucu uccucuagaa augcaugacc cacccugagu uuuggugggc      60

caugcauuuc uagaacuccu uagguaggga auuauccgua uaagaugggu                110


<210>  41
<211>  19
<212>  RNA
<213>  Homo sapiens

<400>  41
ucuagaaaug caugaccca                                                   19

<210>  42
<211>  22
<212>  RNA
<213>  Homo sapiens

<400>  42
acccaccuga ugccccgucc ca                                                22


<210>  43
<211>  110
<212>  RNA
<213>  Homo sapiens

<400>  43
agcauugagg acacaccuug gaggggccug gggaggggca ggaggggugg aaugggcugu        60

uucccuaccc accugaugcc ccgucccagg guugcaaugg cggaggcaga                 110


<210>  44
<211>  110
<212>  RNA
<213>  Homo sapiens

<400>  44
caguuacucu ggcaacagga caagcaaccc aggggaggga aggggagccg aggucaacgc        60

ugcgccucgg ucccuaaccc ccuccggaca accaccgagu ccccuucggu                 110


<210>  45
<211>  108
<212>  RNA
<213>  Homo sapiens

<400>  45
cccgggucuc auugucaacu ggaccaauuu aaccaauuac uauaaaggaa cuauaaagga        60

acauagaaau ugguuaaauu ggagggcuuc cuacagacua auacugga                   108


<210>  46
<211>  110
<212>  RNA
<213>  Homo sapiens

<400>  46
gacaagccac cuaauuugca cccucucccc gcuuuuaacc cuacugccau gaugcaguag        60

gguuaagagu ggggagaaga ugcaaauaac uaccgagccu ugcacauagc                 110


<210>  47
<211>  104
<212>  RNA
<213>  Homo sapiens

<400>  47
uuauuuucua auccgcuguu uuaggguaga cacugacaac guuaugugug gucuuuaacc        60

uguugucaug uuuuuucccu agauguacaa cacugacuca ucau                       104


<210>  48
<211>  21
<212>  RNA
<213>  Homo sapiens

<400>  48

cuucuuagac auggcagcuu c                                                21

<210>  49
<211>  115
<212>  RNA
<213>  Homo sapiens

<400>  49
ccgcuggaag gcuucugggc uugggguuug gugggugacg agauagugag gucccagcuu       60

ugcugcccac aucccucacc cucgacccuc gcuuuccagg cgccugccaa accgc            115

<210>  50
<211>  21
<212>  RNA
<213>  Homo sapiens

<400>  50
   uccucuccuc ccugugccga c                                                21

<210>  51
<211>  100
<212>  RNA
<213>  Homo sapiens

<400>  51
ccaccuuuuu cuucuggucu gcuuguuuca ucuccucuuu guucuguuau ggacacagag       60

gagaugagac aagcagacca gaaaaaauug acuuaauuua                             100

<210>  52
<211>  110
<212>  RNA
<213>  Homo sapiens

<400>  52
gaccccaccu gccagcggug cugccccuuu cucagacccc caugcccagu gcaggcaggg       60

cccuggaaag ggucagcucu cccugacaga gaccagcaga gugaaggacu                  110

<210>  53
<211>  21
<212>  RNA
<213>  Homo sapiens

<400>  53
cggucccuaa cccccuccgg a                                                 21

<210>  54
<211>  110
<212>  RNA
<213>  Homo sapiens

<400>  54
ucucguaucc cagcccacau gcucugcuuc cagugugaca ucgacaggaa uauugaugcc       60

auauuggaag caaggcaugu gggcugggaa aggaggcagu cuuuggaaaa                  110

<210>  55
<211>  21
<212>  RNA
<213>  Homo sapiens

<400>  55

guugucaugu uuuuucccua g                                              21

<210>  56
<211>  110
<212>  RNA
<213>  Homo sapiens

<400>  56
caaucauaau caacuuccua ggcacacuua aaguuauagc uacaucaguu auaacuauau      60

caguuaaaac uuuaagugug ccuaggaagu ugauuuauua cucuucuuca                110

<210>  57
<211>  19
<212>  RNA
<213>  Homo sapiens

<400>  57
ugggcucuca ggggaggug                                                   19

<210>  58
<211>  110
<212>  RNA
<213>  Homo sapiens

<400>  58
aucccugggc uggucugcac aagcucuuag gggcucacuc ugggcuggcc uaaggccaac      60

ugcaggggcu gggcucucag gggaggugag gauggccaca ggucugcaga                110

<210>  59
<211>  22
<212>  RNA
<213>  Homo sapiens

<400>  59
gaccaauuua accaauuacu au                                               22

<210>  60
<211>  111
<212>  RNA
<213>  Homo sapiens

<400>  60
auuauuuacc agcucagaau gugguaggag cuaucagaac uuagugauca agugaagucg      60

uaguuacuaa uuucugaugc ucuuccccug cagaagagag cugugggaag a              111

<210>  61
<211>  110
<212>  RNA
<213>  Homo sapiens

<400>  61
ccuugcacag cacaggauuu ccccaaaccu ugucuggaca uggacuugca ugguccaugu      60

cuagacaagg cuggggaaau ccugcugcua agcaccugga gauguccaca                110

<210>  62
<211>  20
<212>  RNA
<213>  Homo sapiens

<400>  62

ggcuccucag accgccgcag                                                20

<210>  63
<211>  110
<212>  RNA
<213>  Homo sapiens

<400>  63
gcuccagaag accaaccugg agcgcccucg cccggagcgg cggccugcgg gggcacagag      60

cggcuccugg cuccucagac cgccgcagcc cguggcuccu gcccuggguc                110

<210>  64
<211>  113
<212>  RNA
<213>  Homo sapiens

<400>  64
ccucuccggg ccccgugcgg gucggccgug augccucaca cccaagugcu gccucgagca      60

ugcgucuccu gggugagggu gucugagccc acagccccac acccgugguc ccg            113

<210>  65
<211>  22
<212>  RNA
<213>  Homo sapiens

<400>  65
cggccgugau gccucacacc ca                                              22

<210>  66
<211>  101
<212>  RNA
<213>  Homo sapiens

<400>  66
augaguccag uggcucuggu ccauuucccu gccauucccu uggcuucaau uuacucccag      60

ggcuggcagu gacauggguc aaggcucaca ccuucaccug g                         101

<210>  67
<211>  110
<212>  RNA
<213>  Homo sapiens

<400>  67
gucucaggcg cccggggcac cuccgcccuu cuuccugccg cagcuuccuc gcggcacugg      60

gaagggcggc gggaacgcag gcgcugcgcc gggcgaaggc cgcggcccug                110

<210>  68
<211>  19
<212>  RNA
<213>  Homo sapiens

<400>  68
gucuuuugcc cuuucagcu                                                  19

<210>  69
<211>  19
<212>  RNA
<213>  Homo sapiens

<400>  69

ucugcuucca gugugacau                                                19

<210>  70
<211>  110
<212>  RNA
<213>  Homo sapiens

<400>  70
caucauagua agaugugguu uucuguuauc auuguuuuag uguuuguacu gauaaugugg      60

aagcaaacac caaaacaaug aaaacagaaa accacaugau ggguaaaacu                110

<210>  71
<211>  110
<212>  RNA
<213>  Homo sapiens

<400>  71
uccagugccu ggugucucac ugggaaugaa ggggcuggac cucaugguua cugugugccu      60

uucacaggua accaccaugu ccagucccuu cauucccagu gaggcaggcc                110

<210>  72
<211>  19
<212>  RNA
<213>  Homo sapiens

<400>  72
acaccaaaac aaugaaaac                                                  19

<210>  73
<211>  19
<212>  RNA
<213>  Homo sapiens

<400>  73
ggaaugggug acccggccc                                                  19

<210>  74
<211>  110
<212>  RNA
<213>  Homo sapiens

<400>  74
gcagcacagc cggcuccagg cuggaauggg ugacccggcc cuacugaagg aauuucaggg      60

cagggucagc cacucuggcc acugucuccc uccaaugccc ccauacccuc                110

<210>  75
<211>  20
<212>  RNA
<213>  Homo sapiens

<400>  75
gauuucagcg cucugccccu                                                 20

<210>  76
<211>  110
<212>  RNA
<213>  Homo sapiens

<400>  76
uggagcacgu gagugagugc gggaucuggc uggcugcucc gggcacuggc aggagcaagc      60

uccgugucca gaauggccag ccagauccca cacuugcucg cacacuugga 110

<210> 77
<211> 20
<212> RNA
<213> Homo sapiens

<400> 77
cccccuccga gcaggcacug 20

<210> 78
<211> 20
<212> RNA
<213> Homo sapiens

<400> 78
auuucugaug cucuuccccu 20

<210> 79
<211> 110
<212> RNA
<213> Homo sapiens

<400> 79
ggucccucuc cccagcucuc cucccccugg ccccgucgcc ccgcccucgc cgggcugggc 60

ugcgggguca ggggccgagc ggagaggggu gaguauuccc cacagcccuu 110

<210> 80
<211> 105
<212> RNA
<213> Homo sapiens

<400> 80
cuuucccuga uguauaccgu guugugugcc cugagacugc uaaguauuug uauucccaau 60

gccuagcagu cucaggacac acaacagggu guaccuugau gacuu 105

<210> 81
<211> 20
<212> RNA
<213> Homo sapiens

<400> 81
gccccuuucu cagaccccca 20

<210> 82
<211> 19
<212> RNA
<213> Homo sapiens

<400> 82
cuagcagucu caggacaca 19

<210> 83
<211> 110
<212> RNA
<213> Homo sapiens

<400> 83
ggcacuaugg gccaaagaga ccccucuucc cagcacuccc cucugacuuc ccaggccaga 60

ggaggguugc gggaaggggg aucaggcaug ucuggucacc cuggcaaccc 110

<210>  84
<211>  20
<212>  RNA
<213>  Homo sapiens

<400>  84
ccucuuccca gcacuccccu                                                    20


<210>  85
<211>  110
<212>  RNA
<213>  Homo sapiens

<400>  85
agccgacggu gaacggaagu aacccgucag gccuguagag gguggcuggu cccguugcua        60

ugggagcagc aagucucuac gcgccccuca ccgcgacuua aggcuacacu                  110


<210>  86
<211>  21
<212>  RNA
<213>  Homo sapiens

<400>  86
agcaagucuc uacgcgcccc u                                                  21


<210>  87
<211>  19
<212>  RNA
<213>  Homo sapiens

<400>  87
ccgcgcaguc ccggggccc                                                     19


<210>  88
<211>  110
<212>  RNA
<213>  Homo sapiens

<400>  88
ucagggcugc aggagcccac gaguacucga agcccuaugg ggaggcggcg guuagguagu        60

ccgagcgcuc cgcgcagucc cggggccccg cuugguggug acgccgggcc                  110


<210>  89
<211>  110
<212>  RNA
<213>  Homo sapiens

<400>  89
gccccaggau gcaggagauu ugagacccuu ggcaccacug gguaagacuu agcccagaag        60

gagaacagcu cuccuguguc uuggagccuu ucccugacag ugcaaacaga                  110


<210>  90
<211>  110
<212>  RNA
<213>  Homo sapiens

<400>  90
cggugggcac agccacccgg agcggcgcgg gugccgggag cugggcgacg cgccccuccu        60

gccccggcug cgcuguugcc cgggcggccg accuccgugc gugagcgccg                  110

<210>  91
<211>  21
<212>  RNA
<213>  Homo sapiens

<400>  91
gcccagagga ucacggagcc a                                                21


<210>  92
<211>  26
<212>  RNA
<213>  Homo sapiens

<400>  92
cggcgcgggu gccgggagcu gggcga                                           26


<210>  93
<211>  20
<212>  RNA
<213>  Homo sapiens

<400>  93
ccccaggaug caggagauuu                                                  20


<210>  94
<211>  110
<212>  RNA
<213>  Homo sapiens

<400>  94
cugcuggccc uucaggcuac ggugagggug agggugcggg ugaggaugag ggugcgggug      60

aggacccacc cacacccgca cccucacccu cauccucacc cgcagccuca                110


<210>  95
<211>  19
<212>  RNA
<213>  Homo sapiens

<400>  95
uccccgcccu gacacccgc                                                   19


<210>  96
<211>  110
<212>  RNA
<213>  Homo sapiens

<400>  96
ugcaggcugc ugcgguccca gcagccccac ccaccagggu gccaggaggg gccccaggag      60

cacucagcuu ccccgcccug acacccgccc uuuuccccug ccacuuccug                110


<210>  97
<211>  107
<212>  RNA
<213>  Homo sapiens

<400>  97
uuggcucugg ccagguucca gagcuccuug gaucuagugg gaggagucga auggcagggc      60

cccaccauau ccaaggagcc cuggaaccug gccagagcug gguguua                   107


<210>  98

<211>  19
<212>  RNA
<213>  Homo sapiens

<400>  98
gagcagcggc ggugcccac                                                    19


<210>  99
<211>  110
<212>  RNA
<213>  Homo sapiens

<400>  99
cccacccucc ucagcgccca gcgagcagcg gcggugccca cacgcuggug aaccacggcu       60

acgcggagcc cgccgcaggc cgcgagcugc cgcccgacau gaccguggug                 110


<210>  100
<211>  110
<212>  RNA
<213>  Homo sapiens

<400>  100
cucccaucac auccuugcac uccagucuug gugacagagc gagaccuugu cucuuuuuuu       60

ucucgagacc aagucuuaau cugucaucca ggcuggagug cuguggcgcg                 110


<210>  101
<211>  19
<212>  RNA
<213>  Homo sapiens

<400>  101
aaccaccaug uccaguccc                                                    19


<210>  102
<211>  20
<212>  RNA
<213>  Homo sapiens

<400>  102
gugccugugc agagggagcu                                                   20


<210>  103
<211>  101
<212>  RNA
<213>  Homo sapiens

<400>  103
ccucagcugu ugucugggug aggcaucccu gucgugggag cagccacagc ucugccuggu       60

cucccagagc agggacgcuu uguccgcauu uacagcaguc u                          101


<210>  104
<211>  108
<212>  RNA
<213>  Homo sapiens

<400>  104
uucagcugug gcaucugcgg caagagcuuc ucccagcggu cggcccuuau cccccaugcc       60

cgcagccacg cccgggagaa gcccuucaag ugcccugagu gcggcaag                   108


<210>  105

<211>  110
<212>  RNA
<213>  Homo sapiens

<400>  105
agugcgaggg gagggccggg ccgcuggggg ucgagggggu ggggccuggg ccagcgcugg      60

ggaucgggcc uggggccugg auugcgaccc gcggcgggcc gcggcuccua                110


<210>  106
<211>  19
<212>  RNA
<213>  Homo sapiens

<400>  106
uaaaacuuua agugugccu                                                   19


<210>  107
<211>  21
<212>  RNA
<213>  Homo sapiens

<400>  107
cucuccccgc uuuuaacccu a                                                21


<210>  108
<211>  110
<212>  RNA
<213>  Homo sapiens

<400>  108
cgcagaugga gcggggcggc aauggucacc uccgggacuc agcccugugc ugagccccgg      60

gcagugugau cauccuggcc cuucucgugc acguccccug gcuggaugcu                110


<210>  109
<211>  19
<212>  RNA
<213>  Homo sapiens

<400>  109
caagucuuaa ucugucauc                                                   19


<210>  110
<211>  99
<212>  RNA
<213>  Homo sapiens

<400>  110
cacagcagcu cucagauccc cuccaggaau uacaugaugc ccagagagag cauucuggac      60

auuauuuaau uccuggaggg gaucacacug auuguuuga                             99


<210>  111
<211>  20
<212>  RNA
<213>  Homo sapiens

<400>  111
aaaacaggau aggcacuaaa                                                  20


<210>  112
<211>  21
<212>  RNA

<213>  Homo sapiens

<400>  112
cgcaacccac acacggucuc a                                                21


<210>  113
<211>  24
<212>  RNA
<213>  Homo sapiens

<400>  113
gcugggguc gagggggugg ggcc                                              24


<210>  114
<211>  109
<212>  RNA
<213>  Homo sapiens

<400>  114
cuucaucugu uucaccgcua guccccugcu gcauuugugg caguuucaac aaacguuauu       60

ggaaacuucc acggugcagu aggugcuaga cguacaaaga agacaaggc                 109


<210>  115
<211>  110
<212>  RNA
<213>  Homo sapiens

<400>  115
aggcacugcu uguauugugg gugcgcugug gggcacaggc aggaaaugca caagauaacc       60

ccugagcacu uccagccugu guccugccca gcgcaccugc aaggggaggg                 110


<210>  116
<211>  21
<212>  RNA
<213>  Homo sapiens

<400>  116
uagccaauug uccaucuuua g                                                21


<210>  117
<211>  110
<212>  RNA
<213>  Homo sapiens

<400>  117
auuguguacc acagugucua uuuagccaau uguccaucuu uagcuauucu gaaugccuaa       60

agauagacaa uuggcuaaau agaaauugug guacauccau acaauggaau                 110


<210>  118
<211>  19
<212>  RNA
<213>  Homo sapiens

<400>  118
ccgcccuucu uccugccgc                                                   19


<210>  119
<211>  19
<212>  RNA
<213>  Homo sapiens

<400>  119
uggucaccuc cgggacuca                                                    19


<210>  120
<211>  110
<212>  RNA
<213>  Homo sapiens

<400>  120
uccaaacuua uuugucgguc gucucccuug uugcucugac uuuccacuuu guauacccug      60

gggaacugag cacaggaggg aggcgacgcg gcugguguca cuuuggggua                110


<210>  121
<211>  22
<212>  RNA
<213>  Homo sapiens

<400>  121
agaaauuggu uaaauuggag gg                                                22


<210>  122
<211>  19
<212>  RNA
<213>  Homo sapiens

<400>  122
cuuccagccu guguccugc                                                    19


<210>  123
<211>  110
<212>  RNA
<213>  Homo sapiens

<400>  123
ccuaacaucc aucauacagc cagcaaaugg cagagccaga aaaugaaugc aggcagccug      60

acuccugcau ucacauucug gcucugcucu uugcuggcug uaugaugccu                110


<210>  124
<211>  19
<212>  RNA
<213>  Homo sapiens

<400>  124
cuggaagggc aaaagacug                                                    19


<210>  125
<211>  21
<212>  RNA
<213>  Homo sapiens

<400>  125
ugcucuuaca ucucaaacga u                                                 21


<210>  126
<211>  19
<212>  RNA
<213>  Homo sapiens

<400>  126
gggcuucucu cuggccaca                                                    19

<210>  127
<211>  110
<212>  RNA
<213>  Homo sapiens

<400>  127
uaucucuccc acuccaaaug aaucaauccg uguggccaca gagaagcccg cccagcuucu      60

cucugagcug ggcuucucuc uggccacaca aaacauggcu gaauuuguuu               110


<210>  128
<211>  110
<212>  RNA
<213>  Homo sapiens

<400>  128
ggggucguca cuggcgcgga gacgcccccu cuccccccuc ggcucagccg ggcugcugcc      60

cgagcccggg gggugggggg cgucuccccg gcccgucccg uccccggccg               110


<210>  129
<211>  19
<212>  RNA
<213>  Homo sapiens

<400>  129
ccaggaauua caugaugcc                                                  19


<210>  130
<211>  19
<212>  RNA
<213>  Homo sapiens

<400>  130
gcuccuugga ucuagugg g                                                 19


<210>  131
<211>  110
<212>  RNA
<213>  Homo sapiens

<400>  131
gggugcagcu gugaggggga ggggugggga ggagggguac gggagggguu ccccggcucu      60

gacccuggga ugcuucaauc auccuugucc ucuucaaaac accccuguaa               110


<210>  132
<211>  110
<212>  RNA
<213>  Homo sapiens

<400>  132
uggggagagg gagaggggcc augagagggg uguggcgugg ggacgcuccu ccuccucacc      60

ccuguggucc ccucagaucu ucagggucag caaugccaag cuggagccac               110


<210>  133
<211>  24
<212>  RNA
<213>  Homo sapiens

<400>  133
ccacacauag aucuugaagc cacc                                            24

<210>  134
<211>  110
<212>  RNA
<213>  Homo sapiens

<400>  134
ggaggcuaga caugcguccc ugccacacau agaucuugaa gccacccugg uccaggaugu      60

agaaguccug ggagcaaggg uggggcuggu caguaggugg uccagacugg                110


<210>  135
<211>  19
<212>  RNA
<213>  Homo sapiens

<400>  135
gcccgcauuc cuucccucc                                                   19


<210>  136
<211>  110
<212>  RNA
<213>  Homo sapiens

<400>  136
gguuguuguu gccguugugg cugcccgcau uccuucccuc ccaccucgcg gaauuguggc      60

gggaggggga ggaggaaguc agggcgccug cgcggagagg ccgcuuucca                110


<210>  137
<211>  20
<212>  RNA
<213>  Homo sapiens

<400>  137
ucucccagag cagggacgcu                                                  20


<210>  138
<211>  19
<212>  RNA
<213>  Homo sapiens

<400>  138
agaggauugg auugcccua                                                   19


<210>  139
<211>  110
<212>  RNA
<213>  Homo sapiens

<400>  139
aaggauuggc caggggcagc aguaacccag aauagggcaa uccaauuccc uuuccugcuu      60

aggaagggaa gaggauugga uugcccuauu cuggguuaca ugcucacugc                 110


<210>  140
<211>  22
<212>  RNA
<213>  Homo sapiens

<400>  140
agaggagaug agacaagcag ac                                               22


<210>  141
<211>  22

<212>  RNA
<213>  Homo sapiens

<400>  141
cugcuuguuu caucuccucu uu                                                22


<210>  142
<211>  104
<212>  RNA
<213>  Homo sapiens

<400>  142
aggggcucug cacccgcccu ugaggcaccc ucaagcagug gcacgugcug auagguaggc       60

gacagcuuga uggugcgcac gcgggcgucg gcggaggugg ccuc                       104


<210>  143
<211>  21
<212>  RNA
<213>  Homo sapiens

<400>  143
cacgcccggg agaagcccuu c                                                 21


<210>  144
<211>  24
<212>  RNA
<213>  Homo sapiens

<400>  144
ggcgggcagc gggugagggg gugg                                              24


<210>  145
<211>  19
<212>  RNA
<213>  Homo sapiens

<400>  145
cgccccccucu cccccucg                                                    19


<210>  146
<211>  16
<212>  RNA
<213>  Homo sapiens

<400>  146
cauauccaag gagccc                                                       16


<210>  147
<211>  110
<212>  RNA
<213>  Homo sapiens

<400>  147
cccggucacu gagugcgcag gcgccguggg ccucgcccug ccccgcggcg ggaacgcggc       60

cgugagggug ugggguguag guccgccagg ccugcaacgg cugccgaggg                  110


<210>  148
<211>  20
<212>  RNA
<213>  Homo sapiens

<400>  148

ggugggagg agggguacgg                                                20

<210>  149
<211>  20
<212>  RNA
<213>  Homo sapiens

<400>  149
gagaggggug uggcgugggg                                                20

<210>  150
<211>  21
<212>  RNA
<213>  Homo sapiens

<400>  150
caaaccuugu cuggacaugg a                                              21

<210>  151
<211>  110
<212>  RNA
<213>  Homo sapiens

<400>  151
cucuggaugc gugcggggcu cuggccuacc ggugacccgg cuagccggcc gugcuccugc      60

uugagccgcc ugcuggggcc cgcgggccug cugaucucuc gcgcguccga                110

<210>  152
<211>  22
<212>  RNA
<213>  Homo sapiens

<400>  152
cuuccacggu gcaguaggug cu                                             22

<210>  153
<211>  110
<212>  RNA
<213>  Homo sapiens

<400>  153
cagaauuggg gagcugguac uagaggggag gggaauggau gcuggguggg gaaaaugccc      60

acugcauccu ccauccauuc gucccccauc uguccauucu gugaaaccuc                110

<210>  154
<211>  19
<212>  RNA
<213>  Homo sapiens

<400>  154
ugauguccgg caccacccu                                                 19

<210>  155
<211>  110
<212>  RNA
<213>  Homo sapiens

<400>  155
agcugcccgg agcccccagg gcccgugggg augcagggug gagcugguca ggagggcaga      60

gcccggccuu gauguccggc accacccugc aggaaagccu gggcagcagg                110

<210>  156
<211>  20
<212>  RNA
<213>  Homo sapiens

<400>  156
agagcuucuc ccagcggucg                                                    20


<210>  157
<211>  20
<212>  RNA
<213>  Homo sapiens

<400>  157
cagccaauca gcagcgcgga                                                    20


<210>  158
<211>  110
<212>  RNA
<213>  Homo sapiens

<400>  158
cagccaggug agccccggag gcaccgcccc ccguaccgcu gauuggcuga ggguucaccu        60

gccggccaca gccaaucagc agcgcggacc ccuccccagg gcggagcuga                  110


<210>  159
<211>  30
<212>  RNA
<213>  Homo sapiens

<400>  159
uggguguggg cagugggcgg gccaaggaca                                        30


<210>  160
<211>  21
<212>  RNA
<213>  Homo sapiens

<400>  160
ccagaauggc cagccagauc c                                                  21


<210>  161
<211>  19
<212>  RNA
<213>  Homo sapiens

<400>  161
ugcccugaga cugcuaagu                                                     19


<210>  162
<211>  110
<212>  RNA
<213>  Homo sapiens

<400>  162
augcugcagc cucagcugcu cccagcuguc accuucgccc ugcugcccua aguacugaua        60

guccugguag accagggcag gggucgccag cugcaggagu agcagaggcg                  110


<210>  163
<211>  22
<212>  RNA

<213>  Homo sapiens

<400>  163
   agaccgugug uggguugcug ag                                              22

<210>  164
<211>  20
<212>  RNA
<213>  Homo sapiens

<400>  164
uguggggugu agguccgcca                                                  20

<210>  165
<211>  21
<212>  RNA
<213>  Homo sapiens

<400>  165
   cucccuuguu gcucugacuu u                                               21

<210>  166
<211>  21
<212>  RNA
<213>  Homo sapiens

<400>  166
ccuccaucca uucguccccc a                                                21

<210>  167
<211>  21
<212>  RNA
<213>  Homo sapiens

<400>  167
cccccuggcc ccgucgcccc g                                                21

<210>  168
<211>  20
<212>  RNA
<213>  Homo sapiens

<400>  168
gucuguugga ggagggugcg                                                  20

<210>  169
<211>  110
<212>  RNA
<213>  Homo sapiens

<400>  169
uaccugcccu gacugggggc caggcgugcu cuccaucucc agggauucug gggcuugggg      60

aggucauagu cuguuggagg agggugcgcc aauuggccaa aggguguuua               110

<210>  170
<211>  20
<212>  RNA
<213>  Homo sapiens

<400>  170
ccuccgguau ucaagcgauu                                                  20

<210>  171
<211>  110
<212>  RNA
<213>  Homo sapiens

<400>  171
uuccaggguc ccugcacuug gagcauggcc aguguaagug gacaggugu c uggaccaggu      60

gucuguccuc gcacuuggag caugucugcu uacacuggcc auguuccaag      110


<210>  172
<211>  19
<212>  RNA
<213>  Homo sapiens

<400>  172
ccccggcgcg ggcggguuc      19


<210>  173
<211>  19
<212>  RNA
<213>  Homo sapiens

<400>  173
uaucauuguu uuaguguuu      19


<210>  174
<211>  110
<212>  RNA
<213>  Homo sapiens

<400>  174
agcagugacu gcggggugag gggugccugc aucugagggg cucuuugggg agaaacccca      60

cccucccucc cagagaugcu ccucagcccc cacagcucuu ccucuucucu      110


<210>  175
<211>  110
<212>  RNA
<213>  Homo sapiens

<400>  175
uugccuacca cuucaugcug ugaauacaag aauuucuaug ccuguaaucc cagcacaggc      60

auggaaauuc uuguauucac agcaugaagu gguaggcaag ugaggccgag      110


<210>  176
<211>  20
<212>  RNA
<213>  Homo sapiens

<400>  176
cucuccugug ucuuggagcc      20


<210>  177
<211>  110
<212>  RNA
<213>  Homo sapiens

<400>  177
aucaugaaug gcuucagcgc ucgcagcucc ggcgggcggc ggcucggggg cgcgcucggc      60

ucugcucccg gggccguggu ucgcugcggc ugcgagcccg gcccccuccc      110

<210>  178
<211>  21
<212>  RNA
<213>  Homo sapiens

<400>  178
cagcugucac cuucgcccug c                                                21


<210>  179
<211>  110
<212>  RNA
<213>  Homo sapiens

<400>  179
ggggugcaga cucuacauca cagcccugca guuaucacgg gcccauugag gggaggggcc      60

cgugauaacu gcugggcugu gauguagagc cuguacccca guucuggggu                110


<210>  180
<211>  19
<212>  RNA
<213>  Homo sapiens

<400>  180
ugccccugga cagccuggc                                                   19


<210>  181
<211>  110
<212>  RNA
<213>  Homo sapiens

<400>  181
gcacaugugc aggaggcgaa caugccccug gacagccugg ccaucccuuc ucacggcugg      60

ccagcuggcc gggagcaaag ccacagaccc uuucucaaag gccccucuug                110


<210>  182
<211>  23
<212>  RNA
<213>  Homo sapiens

<400>  182
ggccuaccgg ugacccggcu agc                                              23


<210>  183
<211>  21
<212>  RNA
<213>  Homo sapiens

<400>  183
gggcugcggg gucaggggcc g                                                21


<210>  184
<211>  19
<212>  RNA
<213>  Homo sapiens

<400>  184
uucacauucu ggcucugcu                                                   19


<210>  185
<211>  22
<212>  RNA
<213>  Homo sapiens

<400>  185
gacagcuuga uggugcgcac gc                                              22


<210>  186
<211>  21
<212>  RNA
<213>  Homo sapiens

<400>  186
ucgcauugaa ccugagaggc a                                               21


<210>  187
<211>  21
<212>  RNA
<213>  Homo sapiens

<400>  187
gcauggccag uguaagugga c                                               21


<210>  188
<211>  20
<212>  RNA
<213>  Homo sapiens

<400>  188
cugaggggcu cuuuggggag                                                 20


<210>  189
<211>  23
<212>  RNA
<213>  Homo sapiens

<400>  189
aauacaagaa uuucuaugcc ugu                                             23


<210>  190
<211>  20
<212>  RNA
<213>  Homo sapiens

<400>  190
ggggcggagc uccaaccugu                                                 20


<210>  191
<211>  110
<212>  RNA
<213>  Homo sapiens

<400>  191
ccacagcuac aacuggguug cugcagcugc gccagggagg ucggagcucc gcccgcuuaa        60

cucggaaggg ggcggagcuc caaccuguuc cuggcuccca ccagcucccu                  110


<210>  192
<211>  21
<212>  RNA
<213>  Homo sapiens

<400>  192
cccucacccu cgacccucgc u                                               21


<210>  193

<211>  20
<212>  RNA
<213>  Homo sapiens

<400>  193
uagagccuau ccuguuuugc                                                    20


<210>  194
<211>  19
<212>  RNA
<213>  Homo sapiens

<400>  194
cccggggccg ugguucgcu                                                     19


<210>  195
<211>  19
<212>  RNA
<213>  Homo sapiens

<400>  195
ccacacccgc acccucacc                                                     19


<210>  196
<211>  20
<212>  RNA
<213>  Homo sapiens

<400>  196
agcugaggcc uccccgccag                                                    20


<210>  197
<211>  110
<212>  RNA
<213>  Homo sapiens

<400>  197
ccauuaggcg gcugaagcgc gggcggcgcu ggcggcgggg gcugucucgg cuggggcugc        60

ggaggccaag cugaggccuc cccgccagcg uuauuguucg uggagccgcc                  110


<210>  198
<211>  21
<212>  RNA
<213>  Homo sapiens

<400>  198
cugggauccc gcggcgccuc a                                                  21


<210>  199
<211>  110
<212>  RNA
<213>  Homo sapiens

<400>  199
   guuuggggcg gggcgagggc cacugggauc ccgcggcgcc ucaagggccu ugagcuccgc        60
   gggguccccg gcggaucggc cggcccggga gggaaacgga aggaaggcgu                 110


<210>  200
<211>  18
<212>  RNA
<213>  Homo sapiens

<400>  200
ggagggugug gaagacau                                                    18

<210>  201
<211>  19
<212>  RNA
<213>  Homo sapiens

<400>  201
gccauauugg aagcaaggc                                                   19

<210>  202
<211>  19
<212>  RNA
<213>  Homo sapiens

<400>  202
cagagccaga aaaugaaug                                                   19

<210>  203
<211>  20
<212>  RNA
<213>  Homo sapiens

<400>  203
aggguagaca cugacaacgu                                                  20

<210>  204
<211>  21
<212>  RNA
<213>  Homo sapiens

<400>  204
acuucaaguu guuugaccac a                                                21

<210>  205
<211>  110
<212>  RNA
<213>  Homo sapiens

<400>  205
auaaaggaaa agacaaaaag aggcucugug gucaaacaac uugaggugau cagagaauua      60

acugauuacu ucaaguuguu ugaccacaga gccucuuuuu gucuuuuuuu                110

<210>  206
<211>  21
<212>  RNA
<213>  Homo sapiens

<400>  206
cgguugagau gcaagggcug c                                                21

<210>  207
<211>  21
<212>  RNA
<213>  Homo sapiens

<400>  207
ggcccgaucc ccccaccccg g                                                21

<210>  208
<211>  110

<212>  RNA
<213>  Homo sapiens

<400>  208
gcaggcaccg gcagccgauc caggcccgau ccccccaccc cggagccagg ggccguuggg      60

cccuguuguu gggcucgcgg gggugggggg gcucgggccu uuguugguca                110


<210>  209
<211>  21
<212>  RNA
<213>  Homo sapiens

<400>  209
ggggcagcag aggaccuggg c                                               21


<210>  210
<211>  20
<212>  RNA
<213>  Homo sapiens

<400>  210
aagcgcgggg agggaggaua                                                 20


<210>  211
<211>  23
<212>  RNA
<213>  Homo sapiens

<400>  211
ugggguuugg ugggugacga gau                                             23


<210>  212
<211>  110
<212>  RNA
<213>  Homo sapiens

<400>  212
agaagcccgu ucuuucuuac ugccccugga gggagcaugg cgcuagggcu cucagccucc      60

uagaaggaag ggacuaagga gacgggcaca gacuugcguc aucucguuuc               110


<210>  213
<211>  22
<212>  RNA
<213>  Homo sapiens

<400>  213
   gcccccucug ggccagcuga cu                                               22


<210>  214
<211>  110
<212>  RNA
<213>  Homo sapiens

<400>  214
aaggggcuaa auugaagaaa uagcccccuc ugggccagcu gacugggauu ucuacauguu      60

cccagucagu uggccagagg gggcuauuuc uucaauuuag cccuucaugu               110


<210>  215
<211>  22
<212>  RNA
<213>  Homo sapiens

<400>  215
aucaccacca aaccuguucu uc                                                22

<210>  216
<211>  19
<212>  RNA
<213>  Homo sapiens

<400>  216
gcccugcagu uaucacggg                                                    19

<210>  217
<211>  21
<212>  RNA
<213>  Homo sapiens

<400>  217
gguaggagcu aucagaacuu a                                                 21

<210>  218
<211>  110
<212>  RNA
<213>  Homo sapiens

<400>  218
uauauccaga gccugaauga aagagccagu ggugagacag ugaguugauu acuucucacu      60

guuucaccac uggcucuuug guucaugcua acaauguauc ucaccuagau                 110

<210>  219
<211>  24
<212>  RNA
<213>  Homo sapiens

<400>  219
uccccugcug cauuuguggc aguu                                              24

<210>  220
<211>  21
<212>  RNA
<213>  Homo sapiens

<400>  220
ggugagggug ucugagccca c                                                 21

<210>  221
<211>  19
<212>  RNA
<213>  Homo sapiens

<400>  221
cuggcagugg cugcagggc                                                    19

<210>  222
<211>  110
<212>  RNA
<213>  Homo sapiens

<400>  222
gccacagcuc ugcccccguu cccuggcagu ggcugcaggg cagggagagg ucagauuccu      60

cccuguuccc uagugcccgc ucagggugug gccuggccag acugcuggag                 110

<210>  223
<211>  22
<212>  RNA
<213>  Homo sapiens

<400>  223
accaucgacc guugauugua cc                                                22

<210>  224
<211>  21
<212>  RNA
<213>  Homo sapiens

<400>  224
uaccacaggg uagaaccacg g                                                 21

<210>  225
<211>  22
<212>  RNA
<213>  Homo sapiens

<400>  225
ggauaucauc auauacugua ag                                                22

<210>  226
<211>  22
<212>  RNA
<213>  Homo sapiens

<400>  226
aagcugccag uugaagaacu gu                                                22

<210>  227
<211>  21
<212>  RNA
<213>  Homo sapiens

<400>  227
cauuauuacu uuugguacgc g                                                 21

<210>  228
<211>  21
<212>  RNA
<213>  Homo sapiens

<400>  228
acucuuuccc uguugcacua c                                                 21

<210>  229
<211>  21
<212>  RNA
<213>  Homo sapiens

<400>  229
cuagacugaa gcuccuugag g                                                 21

<210>  230
<211>  20
<212>  RNA
<213>  Homo sapiens

<400>  230
ccucugggcc cuuccuccag                                                   20

<210>  231
<211>  22
<212>  RNA
<213>  Homo sapiens

<400>  231
ugagguagua aguuguauug uu                                                22


<210>  232
<211>  22
<212>  RNA
<213>  Homo sapiens

<400>  232
cacccguaga accgaccuug cg                                                22


<210>  233
<211>  22
<212>  RNA
<213>  Homo sapiens

<400>  233
caccuugcgc uacucagguc ug                                                22


<210>  234
<211>  21
<212>  RNA
<213>  Homo sapiens

<400>  234
uaauuuuaug uauaagcuag u                                                 21


<210>  235
<211>  21
<212>  RNA
<213>  Homo sapiens

<400>  235
gcgacccacu cuugguuucc a                                                 21


<210>  236
<211>  22
<212>  RNA
<213>  Homo sapiens

<400>  236
cauugcacuu gucucggucu ga                                                22


<210>  237
<211>  22
<212>  RNA
<213>  Homo sapiens

<400>  237
acugcugagc uagcacuucc cg                                                22


<210>  238
<211>  22
<212>  RNA
<213>  Homo sapiens

<400>  238

cacgcucaug cacacaccca ca                                                22

<210>  239
<211>  22
<212>  RNA
<213>  Homo sapiens

<400>  239
ugagguagua guuuguacag uu                                                22

<210>  240
<211>  22
<212>  RNA
<213>  Homo sapiens

<400>  240
   aauccuuugu cccuggguga ga                                                22

<210>  241
<211>  22
<212>  RNA
<213>  Homo sapiens

<400>  241
caaagaauuc uccuuuuggg cu                                                22

<210>  242
<211>  22
<212>  RNA
<213>  Homo sapiens

<400>  242
agagguagua gguugcauag uu                                                22

<210>  243
<211>  27
<212>  RNA
<213>  Homo sapiens

<400>  243
accuucuugu auaagcacug ugcuaaa                                           27

<210>  244
<211>  21
<212>  RNA
<213>  Homo sapiens

<400>  244
caacaccagu cgaugggcug u                                                 21

<210>  245
<211>  21
<212>  RNA
<213>  Homo sapiens

<400>  245
cucccacaug caggguuugc a                                                 21

<210>  246
<211>  23
<212>  RNA
<213>  Homo sapiens

<400>  246
ucccuguccu ccaggagcuc acg                                              23

<210>  247
<211>  22
<212>  RNA
<213>  Homo sapiens

<400>  247
aacacaccua uucaaggauu ca                                               22

<210>  248
<211>  23
<212>  RNA
<213>  Homo sapiens

<400>  248
guccaguuuu cccaggaauc ccu                                              23

<210>  249
<211>  23
<212>  RNA
<213>  Homo sapiens

<400>  249
ugagcgccuc gacgacagag ccg                                              23

<210>  250
<211>  22
<212>  RNA
<213>  Homo sapiens

<400>  250
cuccugacuc cagguccugu gu                                               22

<210>  251
<211>  22
<212>  RNA
<213>  Homo sapiens

<400>  251
ccuguucucc auuacuuggc uc                                               22

<210>  252
<211>  22
<212>  RNA
<213>  Homo sapiens

<400>  252
cgucaacacu ugcugguuuc cu                                               22

<210>  253
<211>  22
<212>  RNA
<213>  Homo sapiens

<400>  253
uguaaacauc cuugacugga ag                                               22

<210>  254
<211>  23
<212>  RNA
<213>  Homo sapiens

<400> 254
aaugacacga ucacucccgu uga 23

## Description

### TECHNICAL FIELD OF THE INVENTION

The present invention relates to a method for diagnosing and/or prognosing of a disease, preferably lung cancer based on the determination of expression profiles of sets of miRNAs representative for a disease, preferably lung cancer compared to a reference. Furthermore, the present invention relates to sets of polynucleotides and/or primer pairs for detecting sets of miRNAs for diagnosing and/or prognosing of a disease, preferably lung cancer in a biological sample from a subject. Further, the present invention relates to means for diagnosing and/or prognosing of a disease, preferably lung cancer comprising said sets of primer pairs and/or polynucleotides. In addition, the present invention relates to a kit for diagnosing and/or prognosing of a disease, preferably lung cancer comprising means for determining expression profiles of sets of miRNAs representative for a disease, preferably lung cancer and at least one reference. Further, the present invention relates to use of polynucleotides and/or primer pairs for diagnosing and/or prognosing of a disease, preferably lung cancer in a biological samples of a subject. Furthermore, the present invention relates to new miRNA-, miRNA*- and miRNA-precursor sequences identified by next generation sequencing from blood samples.

### BACKGROUND OF THE INVENTION

Today, biomarkers play a key role in early diagnosis, risk stratification, and therapeutic management of various diseases. While progess in biomarker research has accelerated over the last 5 years, the clinical translation of disease biomarkers as endpoints in disease management and as the foundation for diagnostic products still poses a challenge.

MicroRNAs (miRNAs) are a new class of biomarkers. They represent a group of small noncoding RNAs that regulate gene expression at the posttranslational level by degrading or blocking translation of messenger RNA (mRNA) targets. MiRNAs are important players when it comes to regulate cellular functions and in several diseases, including cancer.

So far, miRNAs have been extensively studied in tissue material. It has been found that miRNAs are expressed in a highly tissue-specific manner. Disease-specific expression of miRNAs have been reported in many human cancers employing primarily tissue material as the miRNA source. In this context miRNAs expression profiles were found to be useful in identifying the tissue of origin for cancers of unknown primary origin.

Since recently it is known that miRNAs are not only present in tissues but also in other body fluid samples, including human blood. Nevertheless, the mechanism why miRNAs are found in body fluids, especially in blood, or their function in these body fluids is not understood yet.

Various miRNA biomarkers found in tissue material have been proposed to be correlated with certain diseases, e.g. cancer. However, there is still a need for novel miRNAs as biomarkers for the detection and/or prediction of these and other types of diseases. Especially desirable are non-invasive biomarkers, that allow for quick, easy and cost-effective diagnosis/prognosis which cause only minimal stress for the patient eliminating the need for surgical intervention

Particularly, the potential role of miRNAs as non-invasive biomarkers for the diagnosis and/or prognosis of a disease, preferably lung cancer has not been systematically evaluated yet. In addition, many of the miRNA biomarkers presently available for diagnosing and/or prognosing of diseases have shortcomings such as reduced sensitivity, not sufficient specificity or do not allow timely diagnosis or represent invasive biomarkers. Accordingly, there is still a need for novel and efficient miRNAs or sets of miRNAs as markers, effective methods and kits for the non-invasive diagnosis and/or prognosis of diseases such as a disease, preferably lung cancer.

The inventors of the present invention assessed for the first time the expression of miRNAs on a whole-genome level in subjects with a disease, preferably lung cancer as non-invasive biomarkers from body fluids, preferably in blood. They surprisingly found that miRNAs are significantly dysregulated in blood of a disease, preferably lung cancer subjects in comparison to healthy controls and thus, miRNAs are appropriated non-invasive biomarkers for diagnosing and/or prognosing of a disease, preferably lung cancer. This finding is surprising, since there is nearly no overlap of the miRNA biomarkers found in blood and the miRNA biomarkers found in tissue material representing the origin of the disease. The inventors of the present invention surprisingly found miRNA biomarkers in body fluids, especially in blood, that have not been found to be correlated to a disease, preferably lung cancer when tissues material was used for this kind of analysis. Therefore, the inventors of the invention identified for the first time miRNAs as non-invasive surrogate biomarkers for diagnosis and/or prognosis of a disease, preferably lung cancer. The inventors of the present invention identified single miRNAs which predict a disease, preferably lung cancer with high specificity, sensitivity and accuracy. The inventors of the present invention also pursued a multiple biomarker strategy, thus implementing sets of miRNA biomarkers for diagnosing and/or prognosing of a disease, preferably lung cancer leading to added specificity, sensitivity, accuracy and predictive power, thereby circumventing the limitations of single biomarker. In detail, by using a machine learning algorithms, they identified unique sets of miRNAs (miRNA signatures) that allow for non-invasive diagnosis of a disease, preferably lung cancer with even higher power, indicating that sets of miRNAs (miRNA signatures) derived from a body fluid sample, such as blood from a subject (e.g. human) can be used as novel non-invasive biomarkers.

Furthermore, the inventors of the present invention identified mayor miRNA (miRNA) or minor miRNAs (miRNA*) or miRNA precursor sequences that were previously unknown and were newly identified by next generation sequencing from blood samples of healthy control subjects and lung cancer patients.

### SUMMARY OF THE INVENTION

In a first aspect, the invention provides a method for diagnosing and/or prognosing of a disease, preferably lung cancer comprising the steps of:
(i) determining an expression profile of a set comprising at least two miRNAs representative for a disease, preferably lung cancer in a body fluid sample from a subject, and
(ii) comparing said expression profile to a reference, wherein the comparison of said expression profile to said reference allows for the diagnosis and/or prognosis of a disease, preferably lung cancer,

In a second aspect, the invention provides a set comprising polynucleotides for detecting a set comprising at least two miRNAs for diagnosing and/or prognosing of a disease, preferably lung cancer in a body fluid sample from a subject.

In a third aspect, the invention provides a use of a set of polynucleotides according to the second aspect of the invention for diagnosing and/or prognosing a disease, preferably lung cancer in a subj ect

In a fourth aspect, the invention provides a set of primer pairs for determining the expression level of a set of miRNAs in a body fluid sample of a subject suffering or suspected of suffering from a disease, preferably lung cancer.

In a fifth aspect, the invention provides a use of set of primer pairs according to the fourth aspect of the invention for diagnosing and/or prognosing a disease, preferably lung cancer in a subject In a sixth aspect, the invention provides means for diagnosing and/or prognosing of a disease, preferably lung cancer in a body fluid sample of a subject comprising :
(i) a set of at least two polynucleotides according to the second aspect of the invention or
(ii) a set of primer pairs according the fourth aspect of the invention.

In a seventh aspect, the invention provides a kit for diagnosing and/or prognosing of a disease, preferably lung cancer comprising
(i) means for determining an expression profile of a set comprising at least two miRNAs representative for a disease, preferably lung cancer in a body fluid sample from a subject, and
(ii) at least one reference.

In an eighth aspect, the invention provides a set of miRNAs in a body fluid sample isolated from a subject for diagnosing and/or prognosing of a disease, preferably lung cancer.

In a ninth aspect, the invention provides a use of a set of miRNAs according to the eighth aspect of the invention for diagnosing and/or prognosing of a disease, preferably lung cancer in a subj ect,

This summary of the invention does not necessarily describe all features of the invention.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in this specification and in the appended claims, the singular forms "a", "an", and "the" include plural referents, unless the content clearly dictates otherwise. For example, the term "a test compound" also includes "test compounds".

The terms "microRNA" or "miRNA" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand".

The terms "microRNA*" or "miRNA*" refer to single-stranded RNA molecules of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used. The present invention encompasses (target) miRNAs which are dysregulated in biological samples such as blood or tissue of a disease patients, preferably lung cancer patients in comparison to healthy controls. Said (target) miRNAs are preferably selected from the group consisting of SEQ ID NO: 1 to 222. The term "miRBase" refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download.

As used herein, the term "nucleotides" refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar. Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides of the present invention are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.

In the context of the present invention, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. The present invention encompasses polynucleotides in form of single polynucleotide strands as probes for binding to, hybridizing with or detecting complementary sequences of (target) miRNAs for diagnosing and/or prognosing of a disease, preferably lung cancer. Said (target) miRNAs are preferably selected from the group consisting of SEQ ID NO: 1 to 222.

Because of the conservation of miRNAs among species, for example between humans and other mammals, e.g. animals such as mice, monkey or rat, the polynucleotide(s) of the invention may not only be suitable for detecting a miRNA(s) of a specific species, e.g. a human miRNA, but may also be suitable for detecting the respective miRNA orthologue(s) in another species, e.g. in another mammal, e.g. animal such as mouse or rat.

The term "antisense", as used herein, refers to nucleotide sequences which are complementary to a specific DNA or RNA sequence. The term "antisense strand" is used in reference to a nucleic acid strand that is complementary to the "sense" strand.

The term "label", as used herein, means a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and other entities which can be made detectable. A label may be incorporated into nucleic acids at any position, e.g. at the 3' or 5' end or internally. The polynucleotide for detecting a miRNA (polynucleotide probe) and/or the miRNA itself may be labeled. For detection purposes, the miRNA(s) or miRNA*(s) may be employed unlabeled, directly labeled, or indirectly labeled, such as with biotin to which a streptavidin complex may later bind.

The term "stringent hybridization conditions", as used herein, means conditions under which a first nucleotide sequence (e.g. polynucleotide in its function as a probe for detecting a miRNA or miRNA*) will hybridize to a second nucleotide sequence (e.g. target sequence such as nucleotide sequence of a miRNA or miRNA*), such as in a complex mixture of nucleotide sequences. Stringent conditions are sequence-dependent and will be different in different circumstances. Stringent conditions may be selected to be about 5 to 10°C lower than the thermal melting point (Tm) for the specific sequence at a defined ionic strength, pH. The Tm may be the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01 to1.0 M sodium ion concentration (or other salts) at pH 7.0 to 8.3 and the temperature is at least about 20°C for short probes (e.g. about 10-35 nucleotides) and up to 60°C for long probes (e.g. greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %, Tween 20, 0.1 x TE buffer, 0.5 mg/ml BSA, 0.1 mg/ml herring sperm DNA, incubating at 42°C with wash in 05x SSPE and 6x SSPE at 45°C.

The term "sensitivity", as used herein, means a statistical measure of how well a binary classification test correctly identifies a condition, for example how frequently it correctly classifies a heart and cardiovascular system disease into the correct type out of two or more possible types (e.g. heart and cardiovascular system disease type and healthy type). The sensitivity for class A is the proportion of cases that are determined to belong to class "A" by the test out of the cases that are in class "A". A theoretical, optimal prediction can achieve 100% sensitivity (i.e. predict all patients from the sick group as sick).

The term "specificity", as used herein, means a statistical measure of how well a binary classification test correctly identifies a condition, for example how frequently it correctly classifies a heart and cardiovascular system disease into the correct type out of two or more possible types. The specificity for class A is the proportion of cases that are determined to belong to class "not A" by the test out of the cases that are in class "not A". A theoretical, optimal prediction can achieve 100% specificity (i.e. not predict anyone from the healthy group as sick).

The term "accuracy", as used herein, means a statistical measure for the correctness of classification or identification of sample types. The accuracy is the proportion of true results (both true positives and true negatives).

The term "biological sample", as used in the context of the present invention, refers to any biological sample containing miRNA(s). Said biological sample may be a biological fluid, tissue, cell(s) or mixtures thereof. For example, biological samples encompassed by the present invention are body fluids, tissue (e.g. section or explant) samples, cell culture samples, cell colony samples, single cell samples, collection of single cell samples, blood samples (e.g. whole blood or a blood fraction such as serum or plasma), urine samples, or samples from other peripheral sources. Said biological samples may be mixed or pooled, e.g. a biological sample may be a mixture of blood and urine samples. A "biological sample" may be provided by removing cell(s), cell colonies, an explant, or a section from a subject suspected to be affected by a disease, preferably lung cancer, but may also be provided by using a previously isolated sample. For example, a tissue sample may be removed from a subject suspected to be affected by a disease, preferably lung cancer by conventional biopsy techniques or a blood sample may be taken from a subject suspected to be affected by a disease, preferably lung cancer by conventional blood collection techniques. The biological sample, e.g. tissue or blood sample, may be obtained from a subject suspected to be affected by a disease, preferably lung cancer prior to initiation of the therapeutic treatment, during the therapeutic treatment and/or after the therapeutic treatment.

The term "body fluid sample", as used in the context of the present invention, refers to liquids originating from the body of a subject. Said body fluid samples include, but are not limited to, blood, urine, sputum, breast milk, cerebrospinal fluid, amniotic fluid, bronchial lavage, colostrum, seminal fluid, cerumen (earwax), endolymph, perilymph, gastric juice, mucus, peritoneal fluid, pleural fluid, saliva, sebum (skin oil), semen, sweat, tears, vaginal secretion, vomit including components or fractions thereof. Said body fluid samples may be mixed or pooled, e.g. a body fluid sample may be a mixture of blood and urine samples or blood and tissue material. A "body fluid sample" may be provided by removing a body liquid from a subj ect, but may also be provided by using previously isolated sample material.

Preferably, the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

In the context of the present invention said "body fluid sample" allows for a non-invasive diagnosis/and or prognosis of a subject.

The term "blood sample", as used in the context of the present invention, refers to a blood sample originating from a subject. The "blood sample" may be derived by removing blood from a subject by conventional blood collecting techniques, but may also be provided by using previously isolated and/or stored blood samples. For example a blood sample may be whole blood, plasma, serum, PBMC (peripheral blood mononuclear cells), blood cellular fractions including red blood cells (erythrocytes), white blood cells (leukocytes), platelets (thrombocytes), or blood collected in blood collection tubes (e.g. EDTA-, heparin-, citrate-, PAXgene- , Tempus-tubes) including components or fractions thereof. For example, a blood sample may be taken from a subject suspected to be affected or to be suspected to be affected by a disease, preferably lung cancer, prior to initiation of a therapeutic treatment, during the therapeutic treatment and/or after the therapeutic treatment.

Preferably, the blood sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

In the context of the present invention said "body fluid sample" allows for a non-invasive diagnosis/and or prognosis of a subject.

Preferably, when the blood sample is collected from the subject the RNA-fraction, especially the the miRNA fraction, is guarded against degradation. For this purpose special collection tubes (e.g. PAXgene RNA tubes from Preanalytix, Tempus Blood RNA tubes from Applied Biosystems) or additives (e.g. RNAlater from Ambion, RNAsin from Promega) that stabilize the RNA fraction and/or the miRNA fraction are employed.

The biological sample, preferably the body fluid sample may be from a subject (e.g. human or mammal) that has been therapeutically treated or that has not been therapeutically treated. In one embodiment, the therapeutical treatment is monitored on the basis of the detection of the miRNA or set of miRNAs by the polynucleotide or set of polynucleotides of the invention. It is also preferred that total RNA or a subfraction thereof, isolated (e.g. extracted) from a biological sample of a subject (e.g. human or animal), is used for detecting the miRNA or set of miRNAs by the polynucleotide or set of polynucleotides or primer pairs of the invention.

The term "non-invasive", as used in the context of the present invention, refers to methods for obtaining a biological sample, particularly a body fluid sample, without the need for an invasive surgical intervention or invasive medical procedure. In the context of the present invention, a blood drawn represents a non-invasive procedure, therefore a blood-based test (utilizing blood or fractions thereof) is a non-invasive test. Other body fluid samples for non-invasive tests are e.g. urine, sputum, tears, mothers mild, cerumen, sweat, saliva, vaginal secretion, vomit, etc..

The term "minimal invasive", as used in the context of the present invention, refers to methods for obtaining a biological sample, particularly a body fluid sample, with a minimal need for an invasive surgical intervention or invasive medical procedure.

The term "biomarker", as used in the context of the present invention, represents a characteristic that can be objectively measured and evaluated as an indicator of normal and disease processes or pharmacological responses. A biomarker is a parameter that can be used to measure the onset or the progress of disease or the effects of treatment. The parameter can be chemical, physical or biological.

The term "surrogate biomarker", as used in the context of the present invention, represents biomarker intended to substitute for a clinical endpoint. It is a measure of a clinical condition or a measure of effect of a certain treatment that may correlate with the real clinical condition (e.g. healthy, diseased) but doesn't necessarily have a guaranteed relationship. An ideal surrogate biomarker is a laboratory substitute for a clinically meaningful result, and should lie directly in the causal pathway linking disease to outcome. Surrogate biomarkers are used when the primary endpoint is undesired (e.g. death). A commonly used example is cholesterol : while elevated cholesterol levels increase the likelihood for heart disease, the relationship is not linear - many people with normal cholesterol develop heart disease, and many with high cholesterol do not. "Death from heart disease" is the endpoint of interest, but "cholesterol" is the surrogate biomarker.

The term "diagnosis" as used in the context of the present invention refers to the process of determining a possible disease or disorder and therefore is a process attempting to define the (clinical) condition of a subject. The determination of the expression level of a set of miRNAs according to the present invention correlates with the (clinical) condition of a subject. Preferably, the diagnosis comprises (i) determining the occurrence/presence of a disease, preferably lung cancer, (ii) monitoring the course of a disease, preferably lung cancer, (iii) staging of a disease, preferably lung cancer, (iv) measuring the response of a patient with a disease, preferably lung cancer to therapeutic intervention, and/or (v) segmentation of a subject suffering from a disease, preferably lung cancer.

The term "prognosis" as used in the context of the present invention refers to describing the likelihood of the outcome or course of a disease or a disorder. Preferably, the prognosis comprises (i) identifying of a subject who has a risk to develop a disease, preferably lung cancer, (ii) predicting/estimating the occurrence, preferably the severity of occurrence of a disease, preferably lung cancer, and/or(iii) predicting the response of a subject with a disease, preferably lung cancer to therapeutic intervention.

The term "(clinical) condition" (biological state or health state), as used herein, means a status of a subject that can be described by physical, mental or social criteria. It includes so-called "healthy" and "diseased" conditions. For the definition of "healthy" and "diseased" conditions it is referred to the international classification of diseases (ICD) of the WHO (http://www.int/classifications/icd/en/index.html). When one condition is compared according to a preferred embodiment of the method of the present invention, it is understood that said condition is a disease, preferably lung cancer or a specific form of a disease, preferably lung cancer. When two or more conditions are compared according to another preferred embodiment of the method of the present invention, it is understood that this is possible for all conditions that can be defined and is not limited to a comparison of a diseased versus healthy comparison and extends to multiway comparison, under the proviso that at least one condition is a disease, preferably lung cancers, preferably a specific form of a disease, preferably lung cancer.

The term "miRNA expression profile" as used in the context of the present invention, represents the determination of the miRNA expression level or a measure that correlates with the miRNA expression level in a biological sample. The miRNA expression profile may be generated by any convenient means, e.g. nucleic acid hybridization (e.g. to a microarray, bead-based methods), nucleic acid amplification (PCR, RT-PCR, qRT-PCR, high-throughput RT-PCR), ELISA for quantitation, next generation sequencing (e.g. ABI SOLID, Illumina Genome Analyzer, Roche/454 GS FLX), flow cytometry (e.g. LUMINEX) and the like, that allow the analysis of differential miRNA expression levels between samples of a subject (e.g. diseased) and a control subject (e.g. healthy, reference sample). The sample material measure by the aforementioned means may be total RNA, labeled total RNA, amplified total RNA, cDNA, labeled cDNA, amplified cDNA, miRNA, labeled miRNA, amplified miRNA or any derivatives that may be generated from the aforementioned RNA/DNA species. By determining the miRNA expression profile, each miRNA is represented by a numerical value. The higher the value of an individual miRNA, the higher is the expression level of said miRNA, or the lower the value of an individual miRNA, the lower is the expression level of said miRNA.

The "miRNA expression profile", as used herein, represents the expression level/expression data of a single miRNA or a collection of expression levels of at least two miRNAs, preferably of least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or more, or up to all known miRNAs.

The term "differential expression" of miRNAs as used herein, means qualitative and/or quantitative differences in the temporal and/or local miRNA expression patterns, e.g. within and/or among biological samples, body fluid samples, cells, or within blood. Thus, a differentially expressed miRNA may qualitatively have its expression altered, including an activation or inactivation in, for example, blood from a diseases subject versus blood from a healthy subject. The difference in miRNA expression may also be quantitative, e.g. in that expression is modulated, i.e. either up-regulated, resulting in an increased amount of miRNA, or down-regulated, resulting in a decreased amount of miRNA. The degree to which miRNA expression differs need only be large enough to be quantified via standard expression characterization techniques, e.g. by quantitative hybridization (e.g. to a microarray, to beads), amplification (PCR, RT-PCR, qRT-PCR, high-throughput RT-PCR), ELISA for quantitation, next generation sequencing (e.g. ABI SOLID, Illumina Genome Analyzer, Roche 454 GS FL), flow cytometry (e.g. LUMINEX) and the like.

Nucleic acid hybridization may be performed using a microarray/biochip or *in situ* hybridization. *In situ* hybridization is preferred for the analysis of a single miRNA or a set comprising a low number of miRNAs (e.g. a set of at least 2 to 50 miRNAs such as a set of 2, 5, 10, 20, 30, or 40 miRNAs). The microarray/biochip, however, allows the analysis of a single miRNA as well as a complex set of miRNAs (e.g. a all known miRNAs or subsets therof).

For nucleic acid hybridization, for example, the polynucleotides (probes) according to the present invention with complementarity to the corresponding miRNAs to be detected are attached to a solid phase to generate a microarray/biochip (e.g. 222 polynucleotides (probes) which are complementary to the 222 miRNAs having SEQ ID NO: 1 to 222. Said microarray/biochip is then incubated with a biological sample containing miRNAs, isolated (e.g. extracted) from the body fluid sample such as blood sample from a subject such as a human or an animal, which may be labelled, e.g. fluorescently labelled, or unlabelled. Quantification of the expression level of the miRNAs may then be carried out e.g. by direct read out of a label or by additional manipulations, e.g. by use of a polymerase reaction (e.g. template directed primer extension, MPEA-Assay, RAKE-assay) or a ligation reaction to incorporate or add labels to the captured miRNAs.

Alternatively, the polynucleotides which are at least partially complementary (e.g.a set of chimeric polynucleotides with each a first stretch being complementary to a set of miRNA sequences and a second stretch complementary to capture probes bound to a solid surface (e.g. beads, Luminex beads)) to miRNAs having SEQ ID NO: 1 to 222. are contacted with the biological sample containing miRNAs (e.g a body fluid sample, preferably a blood sample) in solution to hybridize. Afterwards, the hybridized duplexes are pulled down to the surface (e.g a plurality of beads) and successfully captured miRNAs are quantitatively determined (e.g. FlexmiR-assay, FlexmiR v2 detection assays from Luminex).

Nucleic acid amplification may be performed using real time polymerase chain reaction (RT-PCR) such as real time quantitative polymerase chain reaction (RT qPCR). The standard real time polymerase chain reaction (RT-PCR) is preferred for the analysis of a single miRNA or a set comprising a low number of miRNAs (e.g. a set of at least 2 to 50 miRNAs such as a set of 2, 5, 10, 20, 30, or 40 miRNAs), whereas high-throughput RT-PCR technologies (e.g. OpenArray from Applied Biosystems, SmartPCR from Wafergen, Biomark System from Fluidigm) are also able to measure large sets (e.g a set of 10, 20, 30, 50, 80, 100, 200 or more) to all known miRNAs in a high parallel fashion. RT-PCR is particularly suitable for detecting low abandoned miRNAs.

The aforesaid real time polymerase chain reaction (RT-PCR) may include the following steps:
(i) extracting the total RNA from a biological sample or body fluid sample such as a blood sample (e.g. whole blood, serum, or plasma) of a subjects such as human or animal, and obtaining cDNA samples by RNA reverse transcription (RT) reaction using universal or miRNA-specific primers; or collecting a body fluid sample such as urine or blood sample (e.g. whole blood, serum, or plasma) of a patient such as human or animal, and conducting reverse transcriptase reaction using universal or miRNA-specific primers (e.g. looped RT-primers) within the body fluid sample such as urine or blood sample (e.g. whole blood, serum, or plasma) being a buffer so as to prepare directly cDNA samples, (ii) designing miRNA-specific cDNA forward primers and providing universal reverse primers to amplify the cDNA via polymerase chain reaction (PCR), (iii) adding a fluorescent dye (e.g. SYBR Green) or a fluorescent probe (e.g. Taqman probe) probe to conduct PCR, and (iv) detecting the miRNA(s) level in the body fluid sample such as urine or blood sample (e.g. whole blood, serum, or plasma).

A variety of kits and protocols to determine an expression profile by real time polymerase chain reaction (RT-PCR) such as real time quantitative polymerase chain reaction (RT qPCR) are available. For example, reverse transcription of miRNAs may be performed using the TaqMan MicroRNA Reverse Transcription Kit (Applied Biosystems) according to manufacturer's recommendations. Briefly, miRNA may be combined with dNTPs, MultiScribe reverse transcriptase and the primer specific for the target miRNA. The resulting cDNA may be diluted and may be used for PCR reaction. The PCR may be performed according to the manufacturer's recommendation (Applied Biosystems). Briefly, cDNA may be combined with the TaqMan assay specific for the target miRNA and PCR reaction may be performed using ABI7300. Alternative kits are available from Ambion, Roche, Qiagen, Invitrogen, SABiosciences, Exiqon etc.

The term "subject", as used in the context of the present invention, means a patient or individual or mammal suspected to be affected by a disease, preferably lung cancer. The patient may be diagnosed to be affected by a disease, preferably lung cancer, i.e. diseased, or may be diagnosed to be not affected by a disease, preferably lung cancer, i.e. healthy. The subject may also be diagnosed to be affected by a specific form of a disease, preferably lung cancer. The subject may further be diagnosed to develop a disease, preferably lung cancer or a specific form of a disease, preferably lung cancer as the inventors of the present invention surprisingly found that miRNAs representative for a disease, preferably lung cancer are already present in the biological sample, e.g. blood sample, before a disease, preferably lung cancer occurs or during the early stage of a disease, preferably lung cancer. It should be noted that a subject that is diagnosed as being healthy, i.e. not suffering from a disease, preferably lung cancer or from a specific form of a disease, preferably lung cancer, may possibly suffer from another disease not tested/known. The subject may be any mammal, including both a human and another mammal, e.g. an animal such as a rabbit, mouse, rat, or monkey. Human subjects are particularly preferred. Therefore, the miRNA from a subject may be a human miRNA or a miRNA from another mammal, e.g. an animal miRNA such as a mouse, monkey or rat miRNA, or the miRNAs comprised in a set may be human miRNAs or miRNAs from another mammal, e.g. animal miRNAs such as mouse, monkey or rat miRNAs.

The term "control subject", as used in the context of the present invention, may refer to a subject known to be affected with a disease, preferably lung cancer (positive control), i.e. diseased, or to a subject known to be not affected with a disease, preferably lung cancer (negative control), i.e. healthy. It may also refer to a subject known to be effected by another disease/condition (see definition "(clinical) condition"). It should be noted that a control subject that is known to be healthy, i.e. not suffering from a disease, preferably lung cancer, may possibly suffer from another disease not tested/known. The control subject may be any mammal, including both a human and another mammal, e.g. an animal such as a rabbit, mouse, rat, or monkey. Human "control subjects" are particularly preferred.

The term "set comprising at least two miRNAs representative for a disease, preferably lung cancer", as used herein, refers to refers to at least two fixed defined miRNAs comprised in a set which are known to be differential between subjects (e.g. humans or other mammals such as animals) suffering from a disease, preferably lung cancer (diseased state) and control subjects (e.g. humans or other mammals such as animals and are, thus, representative for a disease, preferably lung cancer. Said " set comprising at least two miRNAs representative for a disease, preferably lung cancer" are preferably selected from the group consisting of SEQ ID NO: 1 to 222, a fragment thereof, and a sequence having at least 80% sequence identity thereto.

The term "lung cancer", referrers to carcinoma that forms in tissues of the lung, usually in the cells lining air passages. The two main types are small cell lung cancer and non-small cell lung cancer. Lung cancer may be seen on chest radiograph and computed tomography (CT scan). The diagnosis is confirmed with a biopsy. This is usually performed by bronchoscopy or CT-guided biopsy. Treatment and prognosis depend on the histological type of cancer, the stage (degree of spread), and the patient's general wellbeing, measured by performance status. Common treatments include surgery, chemotherapy, and radiotherapy. NSCLC is sometimes treated with surgery, whereas SCLC usually responds better to chemotherapy and radiation therapy. The non-small-cell lung carcinomas (NSCLC) are grouped together because their prognosis and management are similar. There are three main sub-types: squamous cell lung carcinoma, adenocarcinoma, and large-cell lung carcinoma.

Accounting for 25% of lung cancers, squamous cell lung carcinoma usually starts near a central bronchus. A hollow cavity and associated necrosis are commonly found at the center of the tumor. Well-differentiated squamous cell lung cancers often grow more slowly than other cancer types. Adenocarcinoma accounts for 40% of non-small-cell lung cancers. It usually originates in peripheral lung tissue. Most cases of adenocarcinoma are associated with smoking; however, among people who have never smoked ("never-smokers"), adenocarcinoma is the most common form of lung cancer. A subtype of adenocarcinoma, the bronchioloalveolar carcinoma, is more common in female never-smokers, and may have different responses to treatment.

Due to the shortcomings of current state of the art diagnosis for a disease, preferably lung cancer, there is an urgent need for better, non-invasive tests to further diagnosis and prognosis options for patients.

Due to the shortcomings of current state of the art diagnosis for a disease, preferably lung cancer, there is an urgent need for better, non.invasive tests to further diagnosis and prognosis options for patients.

The inventors of the present invention surprisingly found that miRNAs are significantly dysregulated in body fluid samples such as blood of a disease, preferably lung cancer subjects in comparison to a cohort of controls (healthy subjects) and thus, miRNAs are appropriated biomarkers for diagnosing and/or prognosing of a disease, preferably lung cancer in a non-invasive fashion or minimal-invasive fashion. Furthermore, the sets of miRNAs of the present invention lead to high performance in diagnosing and/or prognosing of a disease, preferably lung cancer, thus expose very high specificity, sensitivity and accuracy. They succeeded in determining the miRNAs that are differentially regulated in body fluid samples from patients having a disease, preferably lung cancer compared to a cohort of controls (healthy subjects) (see experimental section for experimental details). Additionally, the inventors of the present invention performed hypothesis tests (e.g. t-test, limma-test) or other measurements (e.g. AUC, mutual information) on the expression level of the found miRNAs, in all controls (healthy subjects) and subjects suffering from a disease, preferably lung cancer. These tests resulted in a significance value (p-value) for each miRNA. This p-value is a measure for the diagnostic power of each of these single miRNAs to discriminate, for example, between the two clinical conditions: controls (healthy subjects), i.e. not suffering from a disease, preferably lung cancer, or diseased, i.e. suffering from a disease, preferably lung cancer. Since a manifold of tests are carried out, one for each miRNA, the p-values may be too optimistic and, thus, over-estimate the actual discriminatory power. Hence, the p-values are corrected for multiple testing by the Benjamini Hochberg approach.

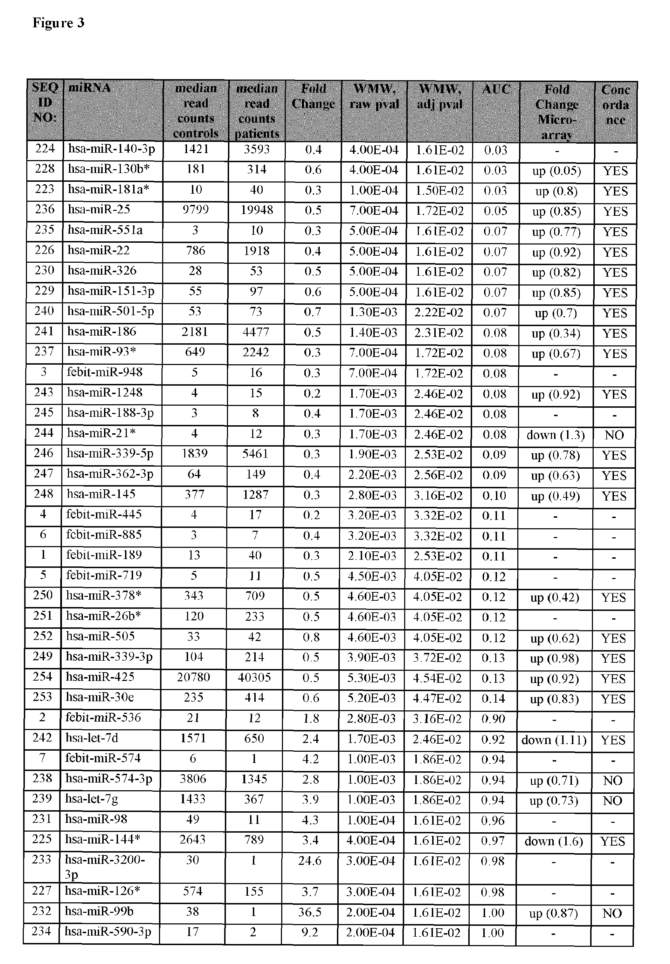

An overview of the miRNAs that are found to be significantly differentially regulated in blood samples of lung cancer patients is provided in Figure 3

Usually the diagnostic power of a single miRNA biomarker is not sufficient to reach high accuracy, specificity and sensitivity for discrimination between healthy subjects (controls) and subjects suffering from a disease, preferably lung cancer, hence no simple threshold method can be used for diagnosis and/or prognosis.

Therefore, the inventors of the present invention employed more than one miRNA biomarker, i.e. sets of miRNA biomarkers (signatures), to further increase and/or improve the performance for diagnosing and/or prognosing of subjects suffering from a disease, preferably lung cancer. This leads to a significant increase in sensitivity, specificity and accuracy when compared to the prior art.

In order to be able to discriminate, for example, between two or more clinical conditions, e.g. healthy and suffering from a disease, preferably lung cancer, for a defined set of miRNA biomarkers, the inventors of the present invention applied a machine learning approach (e.g. t-test, AUC, WMW, support vector machine, hierarchical clustering, or k-means) which leads to an algorithm that is trained by reference data (i.e. data of reference miRNA expression profiles from the two clinical conditions, e.g. healthy and suffering from a disease, preferably lung cancer, for the defined set of miRNA markers) to discriminate between the two statistical classes (i.e. two clinical conditions, e.g. healthy or suffering from a disease, preferably lung cancer).

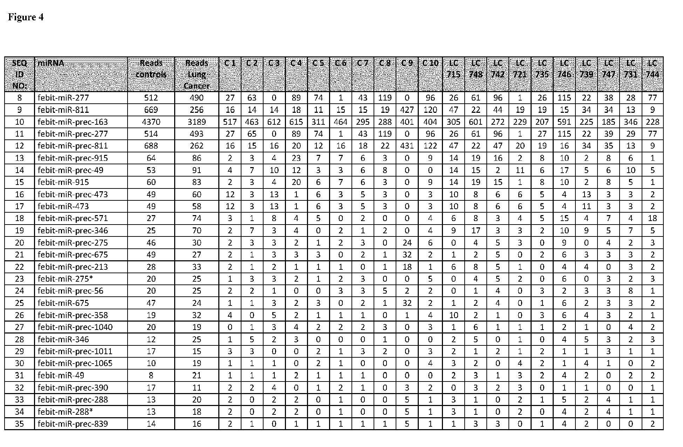

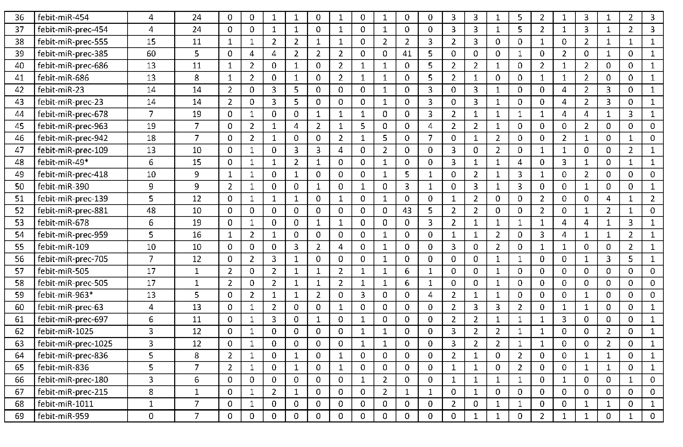

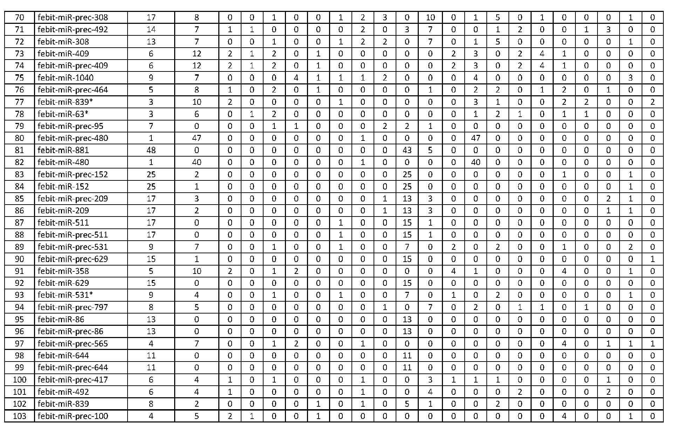

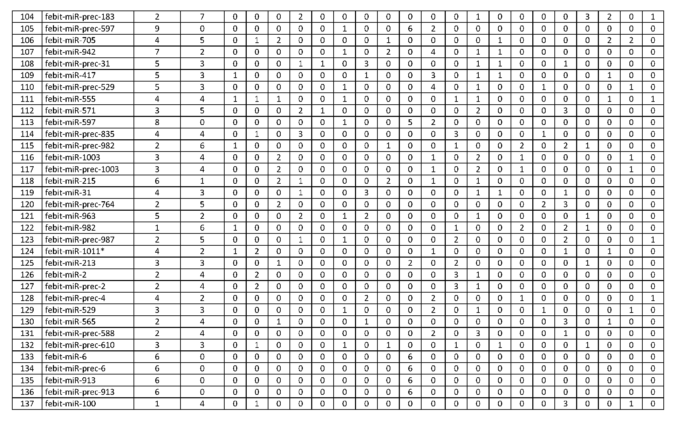

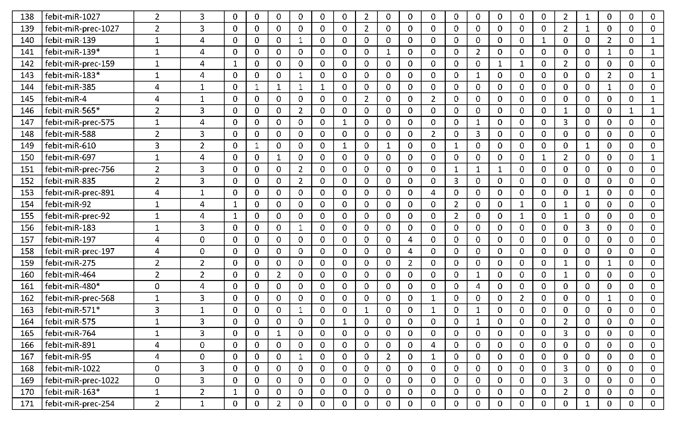

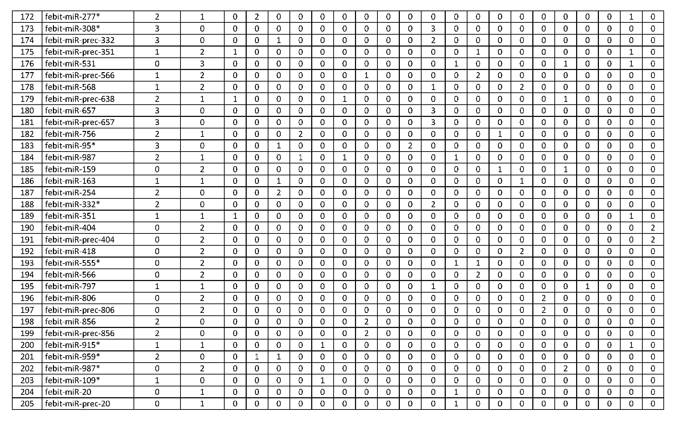

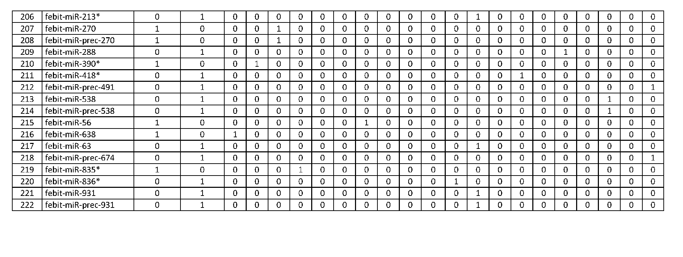

The inventors of the present invention surprisingly found that this approach yields in miRNAs or miRNA sets (signatures) that provide high diagnostic accuracy, specificity and sensitivity in the determination of a disease, preferably lung cancer in patients (see Figure 3 or Figure 4). Said miRNAs or miRNA sets (signatures) comprise at least two miRNAs, wherein the nucleotide sequences of said miRNAs are preferably selected from the group consisting of SEQ ID NO: 1 to 222.

An exemplarily approach to arrive at miRNA sets/signatures that correlate with a disease, preferably lung cancer is summarized below :
- Step 1:: Total RNA (or subfractions thereof) is extracted from the biological sample, e.g. a body fluid sample, preferably a blood sample (including plasma, serum, PBMC or other blood fractions), using suitable kits and/or purification methods.
- Step 2:: From the respective samples the quantity (expression level) of one miRNA or sets of at least two miRNAs, e.g. selected from the group consisting of SEQ ID NO: 1 to SEQ ID NO: 222, is measured using experimental techniques. These techniques include but are not restricted to array based approaches, amplification methods (PCR, RT-PCR, qPCR), sequencing, next generation sequencing, flow cytometry and/or mass spectroscopy.
- Step 3:: In order to gather information on the diagnostic/prognostic value and the redundancy of each of the single miRNA biomarkers, mathematical methods are applied. These methods include, but are not restricted to, basic mathematic approaches (e.g. Fold Quotients, Signal to Noise ratios, Correlation), statistical methods as hypothesis tests (e.g. t-test, Wilcoxon-Mann-Whitney test), the Area under the Receiver operator Characteristics Curve, information theory approaches, (e.g. the Mutual Information, Cross-entropy), probability theory (e.g. joint and conditional probabilities) or combinations and modifications of the previously mentioned methods.
- Step 4:: The information gathered in step 3) is used to estimate for each miRNA biomarker the diagnostic content or value. Usually, however, this diagnostic value is too small to get a highly accurate diagnosis with accuracy rates, specificities and sensitivities beyond the 90% barrier. The diagnostic content of the miRNAs suitable for diagnosing/prognosing a disease, preferably lung cancer is exemplarily listed in Figure 3
- Step 5:: In order to increase the performance for diagnosing/prognosing of subjects suffering from a disease, preferably lung cancer, more than one miRNA biomarker needs to be employed. Thus statistical learning / machine learning / bioinformatics / computational approaches are applied for set selection in order to select/define sets of miRNA biomarkers (e.g. comprising miRNAs SEQ ID NO: 1 to 222) that are tailored for the detection of a disease, preferably lung cancer. These techniques include, but are not restricted to, Wrapper subset selection techniques (e.g. forward step-wise, backward step-wise, combinatorial approaches, optimization approaches), filter subset selection methods (e.g. the methods mentioned in Step 3), principal component analysis, or combinations and modifications of such methods (e.g. hybrid approaches).
- Step 6:: The subsets, selected/defined in Step 5, which may range from only a small number (at least two for the set) to all measured biomarkers is then used to carry out a diagnosis/prognosis of a disease, preferably lung cancer. To this end, statistical learning / machine learning / bioinformatics / computational approaches are applied that include but are not restricted to any type of supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.
- Step 7:: By combination of subset selection (Step 5) and machine learning (Step 6) an algorithm or mathematical function for diagnosing/prognosing a disease, preferably lung cancer is obtained. This algorithm or mathematical function is applied to a miRNA expression profile of a subject to be diagnosed for a disease, preferably lung cancer.

In a first aspect, the present invention relates to a method for diagnosing and/or prognosing of a disease, comprising the steps of:
(i) determining an expression profile of a set comprising at least two miRNAs representative for a disease in a blood sample from a subject, and
(ii) comparing said expression profile to a reference, wherein the comparison of said expression profile to said reference allows for the diagnosis and/or prognosis of a disease,

It is preferred that the body fluid sample is a blood sample, particularly preferred it is a whole blood, a blood cell, a PBMC, a serum, a plasma or a leukocyte or a leukocyte containing sample, more particularly preferred it is whole blood sample containing at least red blood cells, platelets and granulocytes.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs is from the group consisting of SEQ ID NO: 1 to 222

Prefereably the disease to be diagnosed or prognosed is lung cancer, particularly prefererred the disease is non-small-cell lung carcinoma (NSCLC).

When diagnosing and/or prognosing lung cancer it is preferred that further nucleotide sequences of the miRNAs comprised in the set are selected from the group consisting of SEQ ID NO: 223 to 254, a fragment thereof, and a sequence having at least 80% sequence identity thereto.

Thus, it is preferred that the method for diagnosing and/or prognosing of a disease comprises the steps of:
(i) determining an expression profile (expression profile data) of a set comprising, essentially consisting of, or consisting of at least two miRNAs representative for a disease in a blood sample from a subject (e.g. a human or another mammal such as an animal), and
(ii) comparing said expression profile (expression profile data) to a reference, wherein the comparison of said expression profile (expression profile data) to said reference allows for the diagnosis and/or prognosis of a disease.

Thus, for analysis of a body fluid sample (e.g. blood sample) in step (i) of the method of the present invention, an expression profile of a set comprising at least two miRNAs which are known to be differential between subjects (e.g. humans or other mammals such as animals) having or being suspected to have a disease, preferably lung cancer or a special form of a disease, preferably lung cancer (diseased state) and subjects (e.g. humans or other mammals such as animals) not having a disease, preferably lung cancer or a special form of a disease, preferably lung cancer (healthy/control state) and are, thus, representative for a disease, preferably lung cancer, is determined, wherein the nucleotide sequences of said miRNAs are preferably selected from the group consisting of SEQ ID NO: 1 to 222, a fragment thereof, and a sequence having at least 80% sequence identity thereto.

It is more particularly preferred that an expression profile of a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more, or comprising/consisting of 222 miRNAs, representative for a disease, preferably lung cancer in a body fluid sample (e.g. a blood sample) from a subject (e.g. a human or another mammal such as an animal) is determined in the step (i) of the method of the present invention, wherein the nucleotide sequences of said miRNAs are selected from the group consisting of
(i) a nucleotide sequence according to SEQ ID NO: 1 to SEQ ID NO: 222,
(ii) a nucleotide sequence that is a fragment of the nucleotide sequence according to (i), preferably, a nucleotide sequence that is a fragment which is between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the nucleotide sequence according to (i), and
(iii) a nucleotide sequence that has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the nucleotide sequence according to (i) or nucleotide sequence fragment according to (ii).

Additionally, it is more particularly preferred that an expression profile of a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more, or comprising/consisting of 222 miRNAs, representative for a disease, preferably lung cancer in a body fluid sample (e.g. a blood sample) from a subject (e.g. a human or another mammal such as an animal) is determined in the step (i) of the method of the present invention, wherein the set comprising at least two miRNAs is selected from the group consisting of
(i) a set of miRNAs listed in Figure 3
(ii) nucleotide sequences that are fragments of the nucleotide sequence according to (i) or (ii), preferably, nucleotide sequences that are fragments which are between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the nucleotide sequences according to (i) and
(iii) nucleotide sequences that have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the nucleotide sequences according to (i) or (ii)

It is particularly preferred that the nucleotide sequences as defined in (iv) have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20 or more nucleotides, preferably over the whole length, to the nucleotide sequences of the nucleotides according to (i) or (ii) or nucleotide fragments according to (iii).

Furthermore, according to the present invention, a first diagnosis and/or prognosis of a disease, preferably lung cancer can be performed employing, as disclosed, miRNA-detection in a body fluid sample, e.g. in blood , followed by a second diagnosis and/or prognosis that is based on other methods (e.g. other biomarkers and/or imaging methods).

Furthermore, according to the present invention, the set comprising at least two miRNAs for diagnosing and/or prognosing a disease, preferably lung cancer in a body fluid sample, e.g. blood sample, from a patient, e.g. human or animal, may be established on one experimental platform (e.g. microarray/biochip), while for routine diagnosis/prognosis another experimental platform (e.g. qPCR) may be chosen.

Subsequent to the determination of an expression profile (of expression profile data) of a set comprising at least two miRNAs representative for a disease, preferably lung cancer as defined above in a body fluid sample (e.g. blood sample) from a patient (e.g. human or animal) in step (i) of the method for diagnosing and/or prognosing of a disease, preferably lung cancer of the present invention, said method further comprises the step (ii) of comparing said expression profile (expression profile data) to a reference, wherein the comparison of said expression profile (expression profile data) to said reference allows for the diagnosis and/or prognosis of a disease, preferably lung cancer.

The subject (e.g. human or another mammal (e.g. animal)) to be diagnosed with the method of the present invention may suffer, may be suspected to suffer or may not suffer from a disease, preferably lung cancer. The subject (e.g. human or another mammal (e.g. animal)) to be diagnosed with the method of the present invention may suffer from a specific type of a disease, preferably lung cancer. It is also possible to determine, whether the subject (e.g. human or another mammal (e.g. animal) to be diagnosed will develop a disease, preferably lung cancer as the inventors of the present invention surprisingly found that miRNAs representative for a disease, preferably lung cancer are already present in the body fluid sample, e.g. blood sample, before a disease, preferably lung cancer occurs or during the early stage of a disease, preferably lung cancer.

The reference may be the reference (e.g. reference expression profile (data)) of a healthy condition (i.e. not a disease, preferably lung cancer), may be the reference (e.g. reference expression profile (data)) of a diseased condition (i.e. a disease, preferably lung cancer) or may be the reference (e.g. reference expression profiles (data)) of at least two conditions from which at least one condition is a diseased condition (i.e. a disease, preferably lung cancer). For example, (i) one condition may be a healthy condition (i.e. not a disease, preferably lung cancer) and one condition may be a diseased condition (i.e. a disease, preferably lung cancer), or (ii) one condition may be a diseased condition (e.g. a specific form of a disease, preferably lung cancer) and one condition may be another diseased condition (i.e. specific form of a disease, preferably lung cancer, or other timepoint oftreatement, other therapeutic treatment).

Further, the reference may be the reference expression profiles (data) of essentially the same, preferably the same, set of miRNAs of step (i), preferably in a body fluid sample originated from the same source (e.g. urine, or blood such as serum, plasma, or blood cells) as the body fluid sample from the subject (e.g. human or animal) to be tested, but obtained from subjects (e.g. human or animal) known to not suffer from a disease, preferably lung cancer and from subjects (e.g. human or animal) known to suffer from a disease, preferably lung cancer (e.g.a disease, preferably lung cancer, e.g. a disease, preferably lung cancer that has been therapeutically treated).

Preferably, both the reference expression profile and the expression profile of step (i) are determined in the same body fluid sample, e.g. urine, or blood sample including a whole blood, a blood serum sample, blood plasma sample or a blood cell sample (e.g. erythrocytes, leukocytes and/or thrombocytes). It is understood that the reference expression profile is not necessarily obtained from a single subject known to be affected by a disease, preferably lung cancer or known to be not affected by a disease, preferably lung cancer (e.g. healthy subject, such as healthy human or animal, or diseased subject, such as diseased human or animal) but may be an average reference expression profile of a plurality of subjects known to be affected by a disease, preferably lung cancer or known to be not affected by a disease, preferably lung cancer (e.g. healthy subjects, such as healthy humans or animals, or diseased subjects, such as diseased humans or animals), e.g. at least 2 to 200 subjects, more preferably at least 10 to 150 subjects, and most preferably at least 20 to 100 subjects, (e.g. healthy subject, such as healthy human or animal, or diseased subject, such as diseased human or animal). The expression profile and the reference expression profile may be obtained from a subject/patient of the same species (e.g. human or animal), or may be obtained from a subject/patient of a different species (e.g. human or animal). Preferably, the expression profile is obtained from a subject known to be affected by a disease, preferably lung cancer or known to be not affected by a disease, preferably lung cancer of the same species (e.g. human or animal), of the same gender (e.g. female or male) and/or of a similar age/phase of life (e.g. infant, young child, juvenile, adult) as the subject (e.g. human or animal) to be tested or diagnosed.

Thus, in a preferred embodiment of the method of the present invention, the reference is a reference expression profile (data) of at least one subject, preferably the reference is an average expression profile (data) of at least 2 to 200 subjects, more preferably of at least 10 to 150 subjects, and most preferably of at least 20 to 100 subjects, , with one known clinical condition which is a disease, preferably lung cancer or a specific form of a disease, preferably lung cancer, or which is not a disease, preferably lung cancer or a specific form of a disease, preferably lung cancer (i.e. healthy/healthiness), wherein the reference expression profile is the the profile of a set comprising at least two miRNAs that have nucleotide sequences that essentially correspond (are essentially identical), preferably that correspond (are identical), to the nucleotide sequences of the miRNAs of step (i). Preferably, the reference expression profile is the profile of a set comprising at least two miRNAs that have nucleotide sequences that essentially correspond (are essentially identical), preferably that correspond (are identical), to the nucleotide sequences of the miRNAs selected from the group consisting of SEQ ID NO: 1 to 222, a fragment thereof, and a sequence having at least 80% sequence identity thereto of step (i).

The comparison of the expression profile of the patient to be diagnosed (e.g. human or animal) to the (average) reference expression profile may then allow for diagnosing and/or prognosing of a disease, preferably lung cancer or a specific form of a disease, preferably lung cancer (step (ii)), either the subject/patient (e.g. human or animal) to be diagnosed is healthy, i.e. not suffering from a disease, preferably lung cancer, or diseased, i.e. suffering from a disease, preferably lung cancer or a specific form of a disease, preferably lung cancer.

The comparison of the expression profile of the subject (e.g. human or animal) to be diagnosed to said reference expression profile(s) may then allow for the diagnosis and/or prognosis of a disease, preferably lung cancer (step (ii)), either the subject(e.g. human or animal) to be diagnosed is healthy, i.e. not suffering from a disease, preferably lung cancer, or the subject (e.g. human or animal) is diseased, i.e. suffering from a disease, preferably lung cancer.

The comparison of the expression profile of the patient (e.g. human or animal) to be diagnosed to said reference expression profiles may then allow for the diagnosis/prognosis of a specific form of a disease, preferably lung cancer (step (ii)), e.g. whether the patient to be diagnosed suffers from a disease, preferably lung cancer.

In a particularly preferred embodiment of the method of the present invention, the reference is an algorithm or mathematical function. Preferably, the algorithm or mathematical function is obtained on the basis of reference expression profiles (data) of at least 2 to 200 subjects, more preferably of at least 10 to 150 subjects, and most preferably of at least 20 to 100 subjects, with two known clinical conditions from which one is a disease, preferably lung cancer, wherein the reference expression profiles is the profile of a set comprising at least two miRNAs that have nucleotide sequences that essentially correspond (are essentially identical), preferably that correspond (are identical), to the nucleotide sequences of the miRNAs of step (i). Preferably, is the profile of a set comprising at least two miRNAs that have nucleotide sequences that essentially correspond (are essentially identical), preferably that correspond (are identical), to the nucleotide sequences of the miRNAs selected from the group consisting of SEQ ID NO: 1 to 222, a fragment thereof, and a sequence having at least 80% sequence identity thereto of step (i). It is preferred that the algorithm or mathematical function is obtained using a machine learning approach.

Machine learning approaches may include but are not limited to supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.

The inventors of the present invention surprisingly found that the application/use of a machine learning approach (e.g. t-test, AUC, support vector machine, hierarchical clustering, or k-means) leads to the obtainment of an algorithm or mathematical function that is trained by the reference expression profile(s) or reference expression profile data mentioned above (e.g. trained by the miRNA reference expression profile (data) of a diseased condition (i.e. a disease, preferably lung cancer or a specific form of a disease, preferably lung cancer), for example, obtained from subjects (e.g. humans or animals) known to suffer from a disease, preferably lung cancer or from a specific form of a disease, preferably lung cancer (i.e. being diseased) and/or a trained by the miRNA reference expression profile (data) of a healthy condition (i.e. not a disease, preferably lung cancer or a specific form of a disease, preferably lung cancer), for example, obtained from subjects (e.g. humans or animals) known to not suffer from a disease, preferably lung cancer or from a specific form of a disease, preferably lung cancer and that this allows a better (i) discrimination between the at least two (e.g. 2 or 3) clinical conditions (the at least two statistical classes, e.g. the two conditions healthy or suffering from a disease, preferably lung cancer or the two clinical conditions suffering from a specific form of a disease, preferably lung cancer or suffering from another specific form of a disease, preferably lung cancer or at least three clinical conditions, e.g. the three clinical conditions healthy, suffering from a specific form of a disease, preferably lung cancer or suffering from another specific form of a disease, preferably lung cancer or (ii) decision whether the at least one clinical condition (the one condition healthy or suffering from a disease, preferably lung cancer is present. In this way, the performance for diagnosing/prognosing of individuals suffering from a disease, preferably lung cancer can be increased (see also experimental section for details).

Thus, in a preferred embodiment of the method of the present invention, the algorithm or mathematical function is obtained using a machine learning approach, wherein said algorithm or mathematical function is trained by a reference expression profile (data) of at least 2 to 200 subjects, more preferably of at least 10 to 150 subjects, and most preferably of at least 20 to 100 subjects with two known clinical condition for which one is a disease, preferably lung cancer or a specific form of a disease, preferably lung cancer, wherein the reference expression profile is the profile of a set comprising at least two miRNAs that have nucleotide sequences that essentially correspond (are essentially identical), preferably that correspond (are identical), to the nucleotide sequences of the miRNAs of step (i), preferably to decide whether the at least one clinical condition which is a disease, preferably lung cancer or a specific form of a disease, preferably lung cancer.

Further, for instance, the machine learning approach may be applied to the reference expression profiles (data) of a set comprising at least 2 miRNAs (e.g. 10 miRNAs such as miRNAs according to SEQ ID NO: 1 to 10) of at least one subject (e.g. human or animal) known to suffer from a disease, preferably lung cancer and of at least one subject (e.g. human or animal) known to be healthy and may led to the obtainment of an algorithm or mathematical function. This algorithm or mathematical function may then be applied to a miRNA expression profile of the same at least 2 miRNAs as mentioned above (e.g. 10 miRNAs such as miRNAs according to SEQ ID NO: 1 to 10) of a subject (e.g. human or animal) to be diagnosed for a disease, preferably lung cancer and, thus, may then allow to discriminate whether the subject (e.g. human or animal) tested is healthy, i.e. not suffering from a disease, preferably lung cancer, or diseased, i.e. suffering from a disease, preferably lung cancer.

Additionally the algorithm may be trained to discriminate between more than 2 (e.g. 3, 4, 5 or more) clinical conditions from which at least one is a disease, preferably lung cancer.

Preferably, the reference and optionally the expression profile (data) of the miRNA(s) representative for a disease, preferably lung cancer is (are) stored in a database, e.g. an internet database, a centralized, and/or a decentralized database. It is preferred that the reference, e.g. mathematical function or algorithm, is comprised in a computer program, e.g. saved on a data carrier.

The above mentioned method is for diagnosing a disease, preferably lung cancer in a subject, e.g. a human or another mammal such as an animal. Preferably, the diagnosis comprises (i) determining the occurrence/presence of a disease, preferably lung cancer, (ii) monitoring the course of a disease, preferably lung cancer, (iii) staging of a disease, preferably lung cancer, (iv) measuring the response of a patient with a disease, preferably lung cancer to therapeutic intervention, and/or (v) segmentation of a subject suffering from a disease, preferably lung cancer.

Further, the above mentioned method is for prognosis of a disease, preferably lung cancer in a subject, a human or another mammal such as an animal. Preferably, the prognosis comprises (i) identifying of a subject who has a risk to develop a disease, preferably lung cancer, (ii) predicting/estimating the occurrence, preferably the severity of occurrence of a disease, preferably lung cancer, and/or(iii) predicting the response of a subject with a disease, preferably lung cancer to therapeutic intervention.

Further, in a preferred embodiment of the method of the present invention, for determining an expression profile of the set comprising at least two miRNAs representative for a disease, preferably lung cancer in a body fluid sample from a subject comprises a set of miRNAs listed in Figure 3 or Figure 4.

For example, said set comprising 30 miRNAs representative for a disease, preferably lung cancer in a body fluid sample from a subject comprises a set of miRNAs listed in Figure 3 or Figure 4. Alternatively, said set comprising 29, 28, 27 ,26, 25, 24, 23, 22, 21, 20, 19, 18, 17, 16, 15, 14, 13, 12, 11, 10, 9, 8, 7, 6, 5, 4 or 3 miRNAs comprises a set of miRNAs listed in Figure 3 or Figure 4.

For example, said set comprising 30 miRNAs representative for a disease, preferably lung cancer in a body fluid sample from a subject comprises a set of miRNAs listed in Figure 3 or Figure 4. For example, said set comprising 25 miRNAs representative for a disease, preferably lung cancer in a body fluid sample from a subject comprises a set of miRNAs listed in Figure 3 or Figure 4. For example, said set comprising 20 miRNAs representative for a disease, preferably lung cancer in a body fluid sample from a subject comprises a set of miRNAs listed in Figure 3 or Figure 4. For example, said set comprising 15 miRNAs representative for a disease, preferably lung cancer in a body fluid sample from a subject comprises a set of miRNAs listed in Figure 3 or Figure 4. For example, said set comprising 10 miRNAs representative for a disease, preferably lung cancer in a body fluid sample from a subject comprises a set of miRNAs listed in Figure 3 or Figure 4. For example, said set comprising 5 miRNAs representative for a disease, preferably lung cancer in a body fluid sample from a subject comprises a set of miRNAs listed in Figure 3 or Figure 4. Further, in another preferred embodiment of the method of the present invention, for determining an expression profile of the set comprising at least two miRNAs representative for a disease, preferably lung cancer in a body fluid sample from a subject comprises combinations of sets of miRNAs listed in Figure 3 or Figure 4.

For example, said set comprising 30 miRNAs representative for a disease, preferably lung cancer in a body fluid sample from a subject comprises at least 2 sets of miRNAs listed in Figure 3 or

Figure 4. Alternatively, said set comprising 29, 28, 27 ,26, 25, 24, 23, 22, 21, 20, 19, 18, 17, 16, 15, 14, 13, 12, 11, 10, 9, 8, 7, 6, 5 or 4 miRNAs comprises a least 2 sets of miRNAs listed in Figure 3 or Figure 4.

For example, said set comprising 30 miRNAs representative for a disease, preferably lung cancer in a body fluid sample from a subject comprises a least 2 sets of miRNAs listed in Figure 3 or

Figure 4. For example, said set comprising 25 miRNAs representative for a disease, preferably lung cancer in a body fluid sample from a subject comprises a least 2 sets of miRNAs listed in Figure 3 or Figure 4. For example, said set comprising 20 miRNAs representative for a disease, preferably lung cancer in a body fluid sample from a subject comprises a least 2 sets of miRNAs listed in Figure 3 or Figure 4. For example, said set comprising 15 miRNAs representative for a disease, preferably lung cancer in a body fluid sample from a subject comprises a least 2 sets of miRNAs listed in Figure 3 or Figure 4. For example, said set comprising 10 miRNAs representative for a disease, preferably lung cancer in a body fluid sample from a subject comprises a least 2 sets of miRNAs listed in Figure 3 or Figure 4. For example, said set comprising 5 miRNAs representative for a disease, preferably lung cancer in a body fluid sample from a subject comprises a least 2 sets of miRNAs listed in Figure 3 or Figure 4.

In a second aspect, the invention relates to a set comprising polynucleotides for detecting a set comprising at least two miRNAs for diagnosing and/or prognosing of a disease in a body fluid sample from a subject.

It is preferred that the body fluid sample is a blood sample, particularly preferred it is a whole blood, a blood cell, a PBMC, a serum, a plasma or a leukocyte or a leukocyte containing sample, more particularly preferred it is whole blood sample containing at least red blood cells, platelets and granulocytes.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs is from the group consisting of SEQ ID NO: 1 to 222

Prefereably the disease to be diagnosed or prognosed is lung cancer, particularly prefererred the disease is non-small-cell lung carcinoma (NSCLC).

When diagnosing and/or prognosing lung cancer it is preferred that further nucleotide sequences of the miRNAs comprised in the set are selected from the group consisting of SEQ ID NO: 223 to 254, a fragment thereof, and a sequence having at least 80% sequence identity thereto.

It is preferred that the set comprising at least two miRNAs is selected from the miRNAs listed in Figure 3 or Figure 4.

It is preferred that
(i) the polynucleotides comprised in the set of the present invention are complementary to the miRNAs comprised in the set, wherein the nucleotide sequences of said miRNAs are preferably selected from the group consisting of SEQ ID NO: 1 to 222,
(ii) the polynucleotides comprised in the set are fragments of the polynucleotides comprised in the set according to (i), preferably the polynucleotides comprised in the set are fragments which are between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the polynucleotides comprised in the set according to (i), or
(iii) the polynucleotides comprised in the set have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the polynucleotide sequences of the polynucleotides comprised in the set according to (i) or polynucleotide fragments comprised in the set according to (ii).

It is preferred that the polynucleotides of the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 3 5, 40 or more miRNAs, or comprising/consisting of 222 miRNAs and wherein the nucleotide sequences of said miRNAs are selected from the group consisting of SEQ ID NO: 1 to 222.

It is preferred that the polynucleotides of the present invention are for detecting a set comprising, essentially consisting of, or consisting of at least 2 miRNAs, wherein the set comprising, miRNAs is selected from the set listed in Figure 3 or Figure 4.

It is preferred that the polynucleotides of the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40 or more miRNAs, or comprising/consisting of 222 miRNAs and wherein the set of miRNAs comprises at least one of the sets listed in Figure 3 or Figure 4.

For the body fluid sample (e.g. blood sample) analysis, it may be required that a set of polynucleotides (probes) capable of detecting a fixed defined set of miRNAs are attached to a solid support, bead, substrate, surface, platform, or matrix, e.g. biochip, which may be used for body fluid sample (e.g. blood sample) analysis. For example, if the fixed defined set of miRNAs for diagnosing a disease, preferably lung cancer comprises or consists of 20 miRNAs, polynucleotides capable of detecting these 20 miRNAs are attached to a solid support, substrate, surface, platform or matrix, e.g. biochip, in order to perform the diagnostic sample analysis. Alternatively, it may be required that a set of chimeric polynucleotides (probes) capable of detecting a fixed defined set of miRNAs it contacted in solution with a sample containing miRNAs derived from a body fluid sample. The chimeric polynucleotide may comprise of a first sequence stretch that is complementary to a miRNA and a second sequence stretch that allows to pull down the chimeric polynucleotide-miRNA-duplexes to one or more solid supports (e.g. a set of beads for determining the set of miRNAs). For example, a set of 20 chimeric polynucleotides capable of detecting 20 miRNAs are contacted with sample containing miRNAs derived from a body fluid sample in order to form duplexes that can be pulled down to 20 different species of beads and detected theron.

For example, the polynucleotides of the present invention are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 miRNAs wherein the set of miRNAs comprises at least one of the set of miRNAs listed in Figure 3 or Figure 4.

For example, the polynucleotides of the present invention are for detecting a set of 30 miRNAs wherein the set of miRNAs comprises at least one of the sets of miRNAs listed in Figure 3 or Figure 4.

For example, the polynucleotides of the present invention are for detecting a set of 25 miRNAs wherein the set of miRNAs comprises at least one of the sets of miRNAs listed in Figure 3 or Figure 4.

For example, the polynucleotides of the present invention are for detecting a set of 20 miRNAs wherein the set of miRNAs comprises at least one of the sets of miRNAs listed in Figure 3 or Figure 4.

For example, the polynucleotides of the present invention are for detecting a set of 15 miRNAs wherein the set of miRNAs comprises at least one of the sets of miRNAs listed in Figure 3 or Figure 4.

For example, the polynucleotides of the present invention are for detecting a set of 10 miRNAs wherein the set of miRNAs comprises at least one of the sets of miRNAs listed in Figure 3 or Figure 4.

For example, the polynucleotides of the present invention are for detecting a set of 5 miRNAs wherein the set of miRNAs comprises at least one of the sets of miRNAs listed in Figure 3 or Figure 4.

In a third aspect, the invention relates to the use of set of polynucleotides according to the second aspect of the invention for diagnosing and/or prognosing a disease, preferably lung cancer in a subj ect

In a fourth aspect, the invention relates to a set of at least two primer pairs for determining the expression level of a set of miRNAs in a body fluid sample of a subj ect suffering or suspected of suffering from a disease.

It is preferred that the body fluid sample is a blood sample, particularly preferred it is a whole blood, a blood cell, a PBMC, a serum, a plasma or a leukocyte or a leukocyte containing sample, more particularly preferred it is whole blood sample containing at least red blood cells, platelets and granulocytes.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs is from the group consisting of SEQ ID NO: 1 to 222

Prefereably the disease to be diagnosed or prognosed is lung cancer, particularly prefererred the disease is non-small-cell lung carcinoma (NSCLC).

When diagnosing and/or prognosing lung cancer it is preferred that further nucleotide sequences of the miRNAs comprised in the set are selected from the group consisting of SEQ ID NO: 223 to 254, a fragment thereof, and a sequence having at least 80% sequence identity thereto.

It is preferred that the set of at least two primer pairs for determining the expression level of a set of miRNAs in a body fluid sample of a subject suffering or suspected of suffering from a disease are primer pairs that are specific for at least one miRNA listed in Figure 3 or Figure 4.

It is preferred that the set of at least two primer pairs of the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 36, 37, 38, 39, 40 or more miRNAs, or comprising/consisting of 222 miRNAs and wherein the nucleotide sequences of said miRNAs are selected from the group consisting of SEQ ID NO: 1 to 222.

It is preferred that the set of at least two primer pairs of the present invention are for detecting a set comprising, essentially consisting of, or consisting of at least 2 miRNAs, wherein the set comprising, miRNAs is selected from the set listed in Figure 3 or Figure 4.

It is preferred that the set of at least two primer pairs of the present invention are for detecting a set comprising, essentially consisting of, or consisting of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 36, 37, 38, 39, 40 or more miRNAs, or comprising/consisting of 222 miRNAs and wherein the set of miRNAs comprises at least one of the sets listed in Figure 3 or Figure 4.

For example, the set of at least two primer pairs of the present invention are for detecting a set of 40 or 39 or 38 or 37 or 36 or 35 or 34 or 33 or 32 or 31 or 30 or 29 or 28 or 27 or 26 or 25 or 24 or 23 or 22 or 21 or 20 or 19 or 18 or 17 or 16 or 15 or 14 or 13 or 12 or 11 or 10 or 9 or 8 or 7 or 6 or 5 or 4 or 3 or 2 miRNAs wherein the set of miRNAs comprises at least one of the set of miRNAs listed in Figure 3 or Figure 4.

For example, the set of primer pairs of the present invention are for detecting a set of 30 miRNAs wherein the set of miRNAs comprises at least one of the sets of miRNAs listed in Figure 3 or Figure 4. For example, the set of primer pairs of the present invention are for detecting a set of 25 miRNAs wherein the set of miRNAs comprises at least one of the sets of miRNAs listed in Figure 3 or Figure 4. For example, the set of primer pairs of the present invention are for detecting a set of 20 miRNAs wherein the set of miRNAs comprises at least one of the sets of miRNAs listed in Figure 3 or Figure 4. For example, the set of primer pairs of the present invention are for detecting a set of 15 miRNAs wherein the set of miRNAs comprises at least one of the sets of miRNAs listed in Figure 3 or Figure 4. For example, the set of primer pairs of the present invention are for detecting a set of 10 miRNAs wherein the set of miRNAs comprises at least one of the sets of miRNAs listed in Figure 3 or Figure 4.

Preferably, the said primer pairs may be used for amplifying cDNA transcripts of the set of miRNAs selected from the group consisting of SEQ ID 1 to 222. Furthermore, the said primer pairs may be used for amplifying cDNA transcripts of the set of miRNAs listed in Figure 3 or Figure 4

It is understood that the primer pairs for detecting a set of miRNAs may consist of specific and or non-specific primers. Additionally, the set of primer pairs may be complemented by other substances or reagents (e.g. buffers, enzymes, dye, labelled probes) known to the skilled in the art for conducting real time polymerase chain reaction (RT-PCR)

In a fifth aspect, the invention relates to the use of a set of primer pairs according to the fourth aspect of the invention for diagnosing and/or prognosing a disease, preferably lung cancer in a subj ect

In a sixth aspect, the invention relates to means for diagnosing and/or prognosing of a disease, preferably lung cancer in a body fluid sample of a subject.

Preferably, the invention relates to means for diagnosing and/or prognosing of a disease, preferably lung cancer in a body fluid sample of a subject comprising
(i) a set of at least two polynucleotides according to the second aspect of the invention or
(ii) a set of at least two primer pairs according the fourth aspect of the invention.

It is preferred that the body fluid sample is a blood sample, particularly preferred it is a whole blood, a blood cell, a PBMC, a serum, a plasma or a leukocyte or a leukocyte containing sample, more particularly preferred it is whole blood sample containing at least red blood cells, platelets and granulocytes.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs is from the group consisting of SEQ ID NO: 1 to 222

Prefereably the disease to be diagnosed or prognosed is lung cancer, particularly prefererred the disease is non-small-cell lung carcinoma (NSCLC).

When diagnosing and/or prognosing lung cancer it is preferred that further nucleotide sequences of the miRNAs comprised in the set are selected from the group consisting of SEQ ID NO: 223 to 254, a fragment thereof, and a sequence having at least 80% sequence identity thereto.

It is preferred that the set of at least two polynucleotides or the set of at least 2 primer pairs are for detecting a set comprising at least two miRNAs for diagnosing and/or prognosing of a disease, preferably lung cancer in a body fluid sample, e.g. blood sample, from a subject, e.g. patient, human or animal, wherein the set of miRNAs is selected from the miRNAs listed in Figure 3 or Figure 4.

It is preferred that the set of at least two primer pairs for determining the expression level of a set of miRNAs in a body fluid sample of a subject suffering or suspected of suffering from a disease, preferably lung cancer are primer pairs that are specific for at least two miRNAs selected from the group consisting of SEQD ID 1 to 222.

It is preferred that the set of at least two primer pairs for determining the expression level of a set of miRNAs in a body fluid sample of a subject suffering or suspected of suffering from a disease, preferably lung cancer are primer pairs that are specific for at least one set of miRNAs listed in Figure 3 or Figure 4.

It is preferred that the subject is a mammal including both a human and another mammal, e.g. an animal such as a mouse, a rat, a rabbit, or a monkey. It is particularly preferred that the subject is a human.

The present invention provides means for diagnosing and/or prognosing of a disease, preferably lung cancer comprising a set comprising, essentially consisting of, or consisting of at least two polynucleotides (probes) according to the second aspect of the present invention, e.g. a polynucleotide for detecting a set comprising, essentially consisting of, or consisting of at least 2 polynucleotides, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or up to 222 or more polynucleotides for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 or 222 miRNAs or all known miRNAs, wherein the nucleotide sequence of said miRNAs are preferably selected from the group consisting of SEQ ID NO: 1 to 222 , a fragment thereof, and a sequence having at least 80% sequence identity thereto.

The means for diagnosing and/or prognosing of a disease, preferably lung cancer comprises, essentially consists of, or consists of a solid support, substrate, surface, platform or matrix comprising a set comprising, essentially consisting of, or consisting of at least two polynucleotides (probes) according to the second aspect of the present invention, e.g. a solid support, substrate, surface, platform or matrix comprising at least 2 polynucleotides, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more polynucleotides, or comprising/consisting of 222 polynucleotides for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more miRNAs, or comprising/consisting of 222 miRNAs, wherein the nucleotide sequence said miRNAs are preferably selected from the group consisting of SEQ ID NO: 1 to 222, a fragment thereof, and a sequence having at least 80% sequence identity thereto. Preferably, the above mentioned polynucleotide(s) is (are) attached or immobilized to the solid support, substrate, surface, platform or matrix. It is possible to include appropriate controls for non-specific hybridization on the solid support, substrate, surface, platform or matrix.

Additionally, the means for diagnosing and/or prognosing of a disease, preferably lung cancer comprises, essentially consists of, or consists of a solid support, substrate, surface, platform or matrix comprising a set comprising, essentially consisting of, or consisting of at least two polynucleotides (probes) according to the second aspect of the present invention, e.g. a solid support, substrate, surface, platform or matrix comprising at least 2 polynucleotides, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more polynucleotides, or comprising/consisting of 222 polynucleotides for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more miRNAs, or comprising/consisting of 222 miRNAs, wherein the set of miRNAs comprises at least one set of miRNAs listed in Figure 3 or Figure 4. Preferably, the above mentioned polynucleotides are attached or immobilized to the solid support, substrate, surface, platform or matrix. It is possible to include appropriate controls for non-specific hybridization on the solid support, substrate, surface, platform or matrix.

It is particularly preferred that said means for diagnosing and/or prognosing of a disease, preferably lung cancer comprise, essentially consists of, or consists of a microarray/biochip comprising at least two polynucleotides according to the second aspect of the present invention.

It is also preferred that said means for diagnosing and/or prognosing of a disease, preferably lung cancer comprise, essentially consists of, or consists of a set of beads comprising a at least two polynucleotides according to the second aspect of the present invention. It is especially preferred that the beads are employed within a flow cytometer setup or a setup for analysing magnetic beads for diagnosing and/or prognosing of a disease, preferably lung cancer, e.g. in a LUMINEX system (*www.luminexcorp.com)*

Additionally, the present invention provides means for diagnosing and/or prognosing of a disease, preferably lung cancer comprising a set comprising, essentially consisting of, or consisting of at least two primer pairs according to the fourth aspect of the present invention, e.g. of at least 2 primer pairs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 ,39, 40 or up to 222 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 222 miRNAs or all known miRNAs, wherein the nucleotide sequence of said miRNA or the nucleotide sequences of said miRNAs is (are) preferably selected from the group consisting of SEQ ID NO: 1 to 222 , a fragment thereof, and a sequence having at least 80% sequence identity thereto.

Also, the present invention provides means for diagnosing and/or prognosing of a disease, preferably lung cancer comprising a set comprising, essentially consisting of, or consisting of at least two primer pairs according to the fourth aspect of the present invention, e.g. of at least 2 primer pairs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ,37 ,38 ,39, 40 or up to 222 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or 222 miRNAs or all known miRNAs, wherein the set of miRNAs comprises at least one set of miRNAs listed in Figure 3 or Figure 4.

In a seventh aspect, the invention relates to a kit for diagnosing and/or prognosing of a disease, in a subject.

Preferably, the invention relates to a kit for diagnosing and/or prognosing of a disease comprising
(i) means for determining an expression profile of a set comprising at least two miRNAs representative for a disease in a body fluid sample from a subject, and
(ii) at least one reference.

The present invention provides a kit for diagnosing and/or prognosing of a disease, preferably lung cancer comprising
(i) means for determining an expression profile of a a set comprising, essentially consisting of, or consisting of at least two miRNAs (e.g. human miRNAs or miRNAs from another mammal such as an animal (e.g. mouse miRNA or rat miRNAs)), preferably comprising, essentially consisting of, or consisting of at least 2 or up to 222 or more polynucleotides or alternatively a set of at least 2 or up to 222 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ,37, 38 ,39, 40 or more or 222 miRNAs or all known miRNAs, representative for a disease, preferably lung cancer in a biological sample (e.g. a body fluid samples or a blood sample) from a subject (e.g. human or animal), wherein the nucleotide sequence of said miRNA or the nucleotide sequences of said miRNAs is (are) preferably selected from the group consisting of SEQ ID NO: 1 to 222, a fragment thereof, and a sequence having at least 80% sequence identity thereto; and
(ii) at least one reference.

The present invention provides a kit for diagnosing and/or prognosing of a disease, preferably lung cancer comprising
(i) means for determining an expression profile of a a set comprising, essentially consisting of, or consisting of at least two miRNAs (e.g. human miRNAs or miRNAs from another mammal such as an animal (e.g. mouse miRNA or rat miRNAs)), preferably comprising, essentially consisting of, or consisting of at least 2 or up to 222 or more polynucleotides or alternatively a set of at least 2 or up to 222 or more primer pairs for detecting a set comprising, essentially consisting of, or consisting of at least 2 miRNAs, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ,37, 38 ,39, 40 or more or 222 miRNAs or all known miRNAs, representative for a disease, preferably lung cancer in a biological sample (e.g. a body fluid samples or a blood sample) from a subject (e.g. human or animal), wherein the set of miRNAs comprises at least one of the set of miRNAs listed in Figure 3 or Figure 4.
(ii) at least one reference.

Said means may comprise a set comprising, essentially consisting of, or consisting of at least two polynucleotides according to the second aspect of the present invention, a set of at least 2 primer pairs according to the fourth aspect of the invention; means according to the sixth aspect of the present invention; primers suitable to perform reverse transcriptase reaction and/or real time polymerase chain reaction such as quantitative polymerase chain reaction; and/or means for conducting next generation sequencing.

It is particularly preferred that said kit comprises
(ia) a set comprising, essentially consisting of, or consisting of at least two polynucleotides according to the second aspect of the present invention, or a set of primer pairs according to the fourth aspect of the invention and
(ib) optionally at least one of the means selected from the group consisting of: at least one biological sample, for example, tissue sample or body fluid sample, e.g. a blood sample, e.g. whole blood, serum, plasma, or blood cells, of a subject (e.g. human or animal), at least one sample of total RNA extracted from said biological sample, for example, body fluid sample, tissue sample or blood sample, e.g. whole blood, serum, plasma, or blood cells, of a patient (e.g. human or animal), and means to extract RNA from a body fluid sample, e.g. blood sample, e.g. for determining an expression profile of a set comprising, essentially consisting of, or consisting of at least two miRNAs representative for a disease, preferably lung cancer in a body fluid sample (e.g. blood sample) from a patient (e.g. human or animal), wherein the nucleotide sequence of said miRNA or the nucleotide sequences of said miRNAs is (are) preferably selected from the group consisting of SEQ ID NO: 1 to 222, a fragment thereof, and a sequence having at least 80% sequence identity thereto.

It is more particularly preferred that said kit comprises
(ia) a solid support, substrate, surface, platform or matrix (e.g a microarray of a set of beads) according to the third aspect of the present invention comprising a polynucleotide or a set comprising, essentially consisting of, or consisting of at least two polynucleotides according of the first aspect of the present invention, and
(ib) optionally at least one of the means selected from the group consisting of: at least one body fluid sample, for example, tissue or blood sample, e.g. serum, plasma, or blood cells, from a patient (e.g. human or animal), at least one sample of total RNA (or fractions thereof, e.g. miRNA) extracted from a body fluid sample, for example, tissue or blood sample, e.g. serum, plasma, or blood cells, from a patient (e.g. human or animal), means to extract total RNA (or fractions thereof, e.g. miRNA) from a body fluid sample (e.g. blood sample), means for input/injection of a body fluid sample (e.g. blood sample), positive controls for the hybridization experiment, means for holding the solid support, substrate, platform or matrix comprising the polynucleotide(s) (probe(s)), means for labelling the isolated miRNA (e.g. NTP/biotin-NTP), means for hybridization, means to carry out enzymatic reactions (e.g. exonuclease I and/or Klenow enzyme) means for washing steps, means for detecting the hybridization signal, and mean for analysing the detected hybridization signal,e.g. for determining an expression profile of a miRNA or a set comprising, essentially consisting of, or consisting of at least two miRNAs representative for a disease, preferably lung cancer in a body fluid sample (e.g. blood sample) from a patient (e.g. human or animal), wherein the nucleotide sequence of said miRNA or the nucleotide sequences of said miRNAs is (are) preferably selected from the group consisting of SEQ ID NO: 1 to 222, a fragment thereof, and a sequence having at least 80% sequence identity thereto.

Preferably, the above mentioned set comprising, essentially consisting of, or consisting of at least two polynucleotides are attached or immobilized to the solid support, substrate, surface, platform or matrix, e.g. to a microarray or to a set of beads.

Preferably, the above mentioned set comprising, essentially consisting of, or consisting of at least two polynucleotides is (are) attached or immobilized to microarray/biochip.

It is particularly preferred that said kit comprises
(ia) a miRNA-specific primer for reverse transcription of miRNA in miRNA-specific cDNA for a single miRNA (e.g. human miRNA or miRNA from another mammal such as an animal (e.g. mouse or rat miRNA)) or at least two miRNA-specific primers for reverse transcription of miRNAs in miRNA-specific cDNAs for at least 2 miRNAs (e.g. human miRNAs or miRNAs from another mammal such as an animal (e.g. mouse or rat miRNAs)), preferably for at least 3, 4, 5, 6, 7, 8, 9, 10, 11, 12, 13, 14, 15, 16, 17, 18, 19, 20,21,22,23,24,25,26,27,28,29,30,31,32,33,34,35,36,37,38,39,40 or more, or 222 miRNAs (e.g. human miRNAs or miRNAs from another mammal such as an animal (e.g. mouse or rat miRNAs)), comprised in a set of miRNAs, wherein the nucleotide sequence of said miRNA or the nucleotide sequences of said miRNAs is (are) preferably selected from the group consisting of SEQ ID NO: 1 to 222, and
(ib) preferably, a primer set comprising a forward primer which is specific for the cDNA obtained from the miRNA and an universal reverse primer for amplifying the cDNA obtained from the miRNA via real time polymerase chain reaction (RT-PCR) such as real time quantitative polymerase chain reaction (RT qPCR) for the single cDNA obtained from the miRNA or at least two primer sets comprising a forward primer which is specific for the single cDNA obtained from the miRNA and an universal reverse primer for amplifying the cDNA obtained from the miRNA via real time polymerase chain reaction (RT-PCR) such as real time quantitative polymerase chain reaction (RT qPCR) for at least 2, preferably for at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more or 222 cDNAs obtained from the miRNAs comprised in the set of miRNAs, wherein preferably said cDNA is complementary to the nucleotide sequence of the miRNA or said cDNAs are complementary to the nucleotide sequences of the miRNAs selected from the group consisting of SEQ ID NO: 1 to 222, and
(ic) optionally at least one of the means selected from the group consisting of: at least one body fluid sample, for example, tissue or blood sample, e.g. serum, plasma, or blood cells, from a patient (e.g. human or animal), at least one sample of total RNA (or fractions thereof, e.g. miRNA) extracted from a body fluid sample, for example, tissue or blood sample, e.g. serum, plasma, or blood cells, form a patient (e.g. human or animal), means to extract total RNA (or fractions thereof, e.g. miRNA) from a body fluid sample (e.g. blood sample), additional means to carry out the reverse transcriptase reaction (miRNA in cDNA) (e.g. reverse transcriptase (RT) enzyme, puffers, dNTPs, RNAse inhibitor), additional means to carry out real time polymerase chain reaction (RT-PCR) such as real time quantitative PCR (RT qPCR) (e.g. enzymes, puffers, water), means for labelling (e.g. fluorescent label and/or quencher), positive controls for reverse transcriptase reaction and real time PCR, and means for analysing the real time polymerase chain reaction (RT-PCR) result,e.g. for determining an expression profile of a miRNA or a set comprising, essentially consisting of, or consisting of at least 2, preferably comprising, essentially consisting of, or consisting of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more , or 222 miRNAs representative for a disease, preferably lung cancer in a body fluid sample (e.g. blood sample) from a patient (e.g. human or animal), wherein the nucleotide sequence of said miRNA or the nucleotide sequences of said miRNAs is (are) preferably selected from the group consisting of SEQ ID NO: 1 to 222, a fragment thereof, and a sequence having at least 80% sequence identity thereto.

The primer as defined above may also be an oligo-dT primer, e.g. if the miRNA comprises a poly A tail (e.g. as a result of a miRNA elongation, for example, subsequent to RNA extraction) or a miRNA specific looped RT primer (Please amend/adapted if required).

It is also preferred that said kit comprises means for conducting next generation sequencing in order to determine an expression profile of a (single) miRNA or a set comprising, essentially consisting of, or consisting of at least 2 miRNAs representative for a disease, preferably lung cancer in a body fluid sample (e.g. blood sample) from a patient (e.g. human or animal), wherein the nucleotide sequence of said miRNA or the nucleotide sequences of said miRNAs is (are) preferably selected from the group consisting of SEQ ID NO: 1 to 222, a fragment thereof, and a sequence having at least 80% sequence identity thereto. Preferably, said kit further comprises means selected from the group consisting of: at least one body fluid sample, for example, tissue or blood sample, e.g. blood serum, blood plasma, or blood cells from a patient (e.g. human or animal), at least one sample of total RNA (or fractions thereof, e.g. miRNA) extracted from the body fluid sample (e.g. tissue or blood sample) of a patient (e.g. human or animal), and means to extract total RNA (or fractions thereof, e.g. miRNA) from a body fluid sample (e.g. blood sample).

The above mentioned kits further comprise at least one reference (ii). A comparison to said reference may allow for the diagnosis and/or prognosis of a disease, preferably lung cancer. Said reference may be the reference (e.g. reference expression profile (data)) of a healthy condition (i.e. not a disease, preferably lung cancer or a specific form of a disease, preferably lung cancer), may be the reference (e.g. reference expression profile (data)) of a diseased condition (i.e. a disease, preferably lung cancer), or may be the reference (e.g. reference expression (data)) of at least two conditions from which at least one condition is a diseased condition (i.e. a disease, preferably lung cancer).

It is preferred that said reference is a reference expression profile (data) of at least one subject (e.g. human or animal), preferably the reference is an average expression profile (data) of at least 2 to 200 subjects, more preferably at least 10 to 150 subjects, and most preferably at least 20 to 100 subjects, with one known clinical condition which is a disease, preferably lung cancer or a specific form of a disease, preferably lung cancer, or which is not a disease, preferably lung cancer or not a specific form of a disease, preferably lung cancer (i.e. healthy/healthiness), wherein the reference expression profile of a set comprising at least two miRNAs that have nucleotide sequences that essentially correspond (are essentially identical), preferably that correspond (are identical), to the nucleotide sequences of the miRNAs which expression profile is determined by the means of (i).

It is also preferred that said reference are (average) reference expression profiles (data) of at least two subjects, preferably of at least 2 to 200 subjects, more preferably of at least 10 to 150 subjects, and most preferably of at least 20 to 100 subjects, with at least two known clinical conditions, preferably at least 2 to 5, more preferably at least 2 to 4 (i.e. at least 2, 3, 4, or 5) known clinical conditions, from which at least one is a disease, preferably lung cancer), wherein the reference expression profiles are the profiles of a set comprising at least two miRNAs that have nucleotide sequences that essentially correspond (are essentially identical), preferably that correspond (are identical), to the nucleotide sequences of the miRNAs which expression profile is determined by the means of (i).

It is preferred that the reference is generated from expression profilies (data) obtained from 2 clinical conditions, which are a disease, preferably lung cancer and healthy control.

Preferably, (i) the (average) reference expression profile (data), which is provided with the kit, is determined in the same type of body fluid sample (e.g. blood and/or urine sample) and/or obtained from (control) subject(s) of the same species, gender and/or of similar age/stage of life, or (ii) the (average) reference expression profiles (data), which are provided with the kit, are determined in the same type of body fluid sample (e.g. blood and/or urine sample) and/or are obtained from (control) subject(s) of the same species, gender and/or of similar age/stage of life.

Said reference, preferably said (average) reference expression profile(s) (data) may be comprised in an information leaflet (e.g. for comparing tested single reference miRNA biomarkers with the expression profile data of a patient to be diagnosed) or saved on a data carrier (e.g. for comparing tested sets of miRNA biomarkers with the expression profile data of a patient to be diagnosed). Said reference, preferably said (average) reference expression profile(s) (data) may also be comprised in a computer program which is saved on a data carrier. The kit may alternatively comprise an access code which allows the access to a database, e.g. an internet database, a centralized or a decentralized database, where said reference, preferably said (average) reference expression profile(s) (data) is (are) comprised.

It is particularly preferred that the reference is an algorithm or mathematical function.

Preferably the algorithm or mathematical function is obtained from a reference expression profile (data) of at least one subject, preferably the algorithm or mathematical function is obtained from an average reference expression profile (data) of at least 2 to 200 subjects, more preferably of at least 10 to 150 subjects, and most preferably of at least 20 to 100 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with one known clinical condition which is a disease, preferably lung cancer or a specific form of a disease, preferably lung cancer, or which is not a disease, preferably lung cancer or a specific form of a disease, preferably lung cancer (i.e. healthy/healthiness), wherein the reference expression profile is the profile of a single miRNA that has a nucleotide sequence that essentially corresponds (is essentially identical), preferably that corresponds (is identical), to the nucleotide sequence of the miRNA which expression profile is determined by the means of (i), or is the profile of a set comprising at least two miRNAs that have nucleotide sequences that essentially correspond (are essentially identical), preferably that correspond (are identical), to the nucleotide sequences of the miRNAs which expression profile is determined by the means of (i).

It is also preferred that the algorithm or mathematical function is obtained from (average) reference expression profiles (data) of at least two subjects, preferably of at least 2 to 200 subjects, more preferably of at least 10 to 150 subjects, and most preferably of at least 20 to 100 subjects, i.e. of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least two known clinical conditions, preferably at least 2 to 5, more preferably at least 2 to 4 (i.e. at least 2, 3, 4, or 5) known clinical conditions, from which at least one is a disease, preferably lung cancer, wherein the reference expression profiles are the profiles of a single miRNA that has a nucleotide sequence that essentially corresponds (is essentially identical), preferably that corresponds (is identical), to the nucleotide sequence of the miRNA which expression profile is determined by the means of (i) or are the profiles of a set comprising at least two miRNAs that have nucleotide sequences that essentially correspond (are essentially identical), preferably that correspond (are identical), to the nucleotide sequences of the miRNAs which expression profile is determined by the means of (i).

It is preferred that the algorithm or mathematical function is obtained using a machine learning approach (see second aspect of the present invention).

Preferably, the algorithm or mathematical function is saved on a data carrier comprised in the kit or the computer program, wherein the algorithm or mathematical function is comprised, is saved on a data carrier comprised in the kit. Said kit may alternatively comprise an access code which allows the access to an internet page, where the algorithm or mathematical function is saved or where the computer program, wherein the algorithm or mathematical function is comprised, can be downloaded.

Preferably, the algorithm or mathematical function is saved on a data carrier or the algorithm or mathematical function is comprised in a computer program which is saved on a data carrier. Said kit may alternatively comprise an access code which allows the access to a database or an internet page, where the algorithm or mathematical function is comprised, or where a computer program comprising the algorithm or mathematical function can be downloaded.

More than one reference may be comprised in the kit, e.g. 2, 3, 4, 5, or more references. For example, the kit may comprise reference data, preferably (average) reference expression profile(s) (data), which may be comprised in an information leaflet or saved on a data carrier. In addition, the kit may comprise more than one algorithm or mathematical function, e.g. two algorithms or mathematical functions, e.g. one trained to discriminate between a healthy condition and a disease, preferably lung cancer and one trained to discriminate between specific forms of a disease, preferably lung cancer, e.g. comprised in a computer program, preferably stored on a data carrier.

In an eighth aspect, the invention relates to a set of miRNAs isolated from a body fluid sample from a subject for diagnosing and/or prognosing of a disease, wherein the miRNAs are selected from the group consisting of SEQ ID 1 to 222.

It is preferred that the body fluid sample is a blood sample, particularly preferred it is a whole blood, a blood cell, a PBMC, a serum, a plasma or a leukocyte or a leukocyte containing sample, more particularly preferred it is whole blood sample containing at least red blood cells, platelets and granulocytes.

It is preferred that the subject is a mammal including both a human and another mammal, e.g. an animal such as a mouse, a rat, a rabbit, or a monkey. It is particularly preferred that the subject is a human.

Preferably, the set comprising at least two miRNAs is from the group consisting of SEQ ID NO: 1 to 222

Prefereably the disease to be diagnosed or prognosed is lung cancer, particularly prefererred the disease is non-small-cell lung carcinoma (NSCLC).

When diagnosing and/or prognosing lung cancer it is preferred that further nucleotide sequences of the miRNAs comprised in the set are selected from the group consisting of SEQ ID NO: 223 to 254, a fragment thereof, and a sequence having at least 80% sequence identity thereto.

Preferably, the predetermined set comprising at least two miRNAs that are differentially regulated in blood samples from a disease patients as compared to healthy controls is selected from the miRNAs listed in Figure 3 or Figure 4.

It is preferred that the predetermined set comprising at least two miRNAs that are differentially regulated in blood samples from a disease, preferably lung cancer patients as compared to healthy controls comprises at least one miRNA listed in Figure 3 or Figure 4

In a ninth aspect, the invention relates to the use of a set of miRNAs according to the eighth aspect of the invention for diagnosing and/or prognosing of a disease, preferably lung cancer in a subj ect,

In a further aspect, the present invention relates to a method for diagnosing and/or prognosing of a disease, preferably lung cancer comprising the steps of:
(i) providing a set comprising at least two polynucleotides according to the second aspect of the present invention for detecting a set comprising at least two miRNAs representative for a disease, preferably lung cancer in a body fluid sample (e.g. blood sample) from a patient (e.g. human or animal),
   wherein the nucleotide sequence of said miRNA or the nucleotide sequences of said miRNAs is (are) preferably selected from the group consisting of SEQ ID NO: 1 to 222, a fragment thereof, and a sequence having at least 80% sequence identity thereto,
(ii) using the polynucleotide(s) provided in (i) for determining an miRNA expression profile in a body fluid sample (e.g. blood sample) from a patient (e.g. human or animal) with an unknown clinical condition,
(iii) comparing said expression profile to a reference,
(iv) diagnosing or prognosing the clinical condition of the patient (e.g. human or animal) on the basis of said comparison.

The term "patient with an unknown clinical condition" refers to a patient (e.g. human or animal) which may suffer from a disease, preferably lung cancer (i.e. diseased patient) or may not suffer from a disease, preferably lung cancer (i.e. healthy patient). The patient (e.g. human or animal) to be diagnosed may further suffer from a specific type of a disease, preferably lung cancer. It is also possible to determine, whether the patient (e.g. human or animal) to be diagnosed will develop the above mentioned disease as the inventors of the present invention surprisingly found that miRNAs representative for a disease, preferably lung cancer are already present in the body fluid sample, e.g. blood sample, before a disease, preferably lung cancer occurs or during the early stage of a disease, preferably lung cancer. It should be noted that a patient that is diagnosed as being healthy, i.e. not suffering from a disease, preferably lung cancer, may possibly suffer from another disease not tested/known.

In a further aspect, the present invention relates to new nucleotide sequences for non-invasive diagnosis and/or prognosis of diseases. The nucleotide sequences for non-invasive diagnosis and/or prognosis of diseases are selected from the group consisting of SEQ ID NO: 1-222. The non-invasive diagnosis and/or prognosis of diseases according to the present invention is based on the analysis of nucleotide sequences selected from the group consisting of SEQ ID NO: 1-222 in a body fluid sample of a subject, prefereably in a blood sample, particularly preferred in a whole blood, a blood cell, a PBMC, a serum, a plasma or a leukocyte or a leukocyte containing sample, more particularly preferred in whole blood sample containing at least red blood cells, platelets and granulocytes.

The present invention further relates to an isolated nucleic acid molecule selected from the group consisting of
(a) a nucleotide sequence shown in SEQ ID NO: 1-222
(b) a nucleotide sequence which is the complement of (a),
(c) a nucleotide sequence which is the DNA complement of (a) or (b), and
(d) a nucleotide sequence which has a sequence identity of at least 90% to (a) or (b) or (c)

The present invention further relates to an isolated nucleic acid molecule selected from the group consisting of
(a) a nucleotide sequence shown in SEQ ID NO: 10-14, 16, 18-22, 24, 26-27. 29-30, 32-33, 35, 37-40, 43-47, 49, 51-52, 54, 56, 58, 60-61, 63-64, 66-67, 70-71, 74, 76, 79-80, 83, 85, 88-90, 94, 96-97, 99-100, 103-105, 108, 110, 114-115, 117, 120, 123, 127-128, 131-132, 134, 136, 139 142, 147, 151, 153, 155, 158, 162, 169, 171, 174-175, 177, 179, 181, 191, 197, 199, 205, 208, 212, 214, 218, 223
(b) a nucleotide sequence of 16-32 nucleotides which is a fragment of the nucleotide sequence of (a),
(c) a nucleotide sequence which is the complement of (a) or (b)
(d) a nucleotide sequence which is the DNA complement of (a) or (b) or (c), and
(e) a nucleotide sequence which has a sequence identity of at least 90% to (a) or (b) or (c) or (d)

In summary, the present invention is composed of the following items :
1. A method for diagnosing and/or prognosing of a disease comprising the steps of:
   (i) determining an expression profile of a set comprising at least two miRNAs representative for the disease in a blood sample from a subject, and
   (ii) comparing said expression profile to a reference, wherein the comparison of said expression profile to said reference allows for the diagnosis and/or prognosis of the disease,
   wherein at least one nucleotide sequences of the miRNAs comprised in the set is selected from the group consisting of SEQ ID NO: 1 to 222, a fragment thereof, and a sequence having at least 90% sequence identity thereto.
2. The method of item 1, wherein the blood sample is a whole blood sample containing red blood cells, platelets and granulocytes
3. The method of any of the items 1 or 2, wherein the disease is lung cancer
4. The method of item 3, wherein further nucleotide sequences of the miRNAs comprised in the set are selected from the group consisting of SEQ ID NO: 223 to 254, a fragment thereof, and a sequence having at least 80% sequence identity thereto.
5. A set comprising polynucleotides for detecting a set comprising at least two miRNAs for diagnosing and/or prognosing of a disease in a blood sample from a subject, wherein at least one nucleotide sequences of the miRNAs comprised in the set is selected from the group consisting of SEQ ID NO: 1 to 222.
6. The set comprising polynucleotides of item 5, wherein the blood sample is a whole blood sample containing red blood cells, platelets and granulocytes
7. The set comprising polynucleotides of any of the items 5 or 6, wherein the disease is lung cancer
8. The set comprising polynucleotides of item 7, wherein further nucleotide sequences of the miRNAs comprised in the set are selected from the group consisting of SEQ ID NO: 223 to 254
9. The set comprising polynucleotides according to any of the items 5 to 8, wherein
   (i) the polynucleotides comprised in the set are complementary to the miRNAs comprised in the set according to items 5 or 8,
   (ii) the polynucleotides comprised in the set are fragments of the polynucleotides comprised in the set according to (i), or
   (iii) the polynucleotides comprised in the set have at least 90% sequence identity to the polynucleotide sequences of the polynucleotides comprised in the set according to (i) or polynucleotide fragments comprised in the set according to (ii).
10. Use of set of polynucleotides according to any of the items 5 to 9 for diagnosing and/or prognosing of a disease in a subject
11. Use of set of polynucleotides according to item 10, wherein the disease is lung cancer
12. Means for diagnosing and/or prognosing of a disease in a blood sample of a subject comprising :
   (i) A set comprising polynucleotides for detecting a set comprising at least two miRNAs according to any of the items 5 to 9 and
   (ii) a biochip, a RT-PCT system, a PCR-system, a flow cytometer, Luminex system or a next generation sequencing system.
13. Means for diagnosing and/or prognosing according to item 12, wherein the disease is lung cancer
14. A kit for diagnosing and/or prognosing of a disease comprising
   (i) means for determining an expression profile of a set comprising at least two miRNAs representative for the disease in a blood sample from a subject according to any of the items 12 or 13, and
   (ii) at least one reference.
15. A set of at least 2 miRNAs isolated from a blood sample from a subject for diagnosing and/or prognosing of a disease, wherein at least one miRNA is selected from the group consisting of SEQ ID NO: 1 to 222
16. The set of miRNAs of item 15, wherein the blood sample is a whole blood sample containing red blood cells, platelets and granulocytes
17. The set of miRNAs of any of the items 15 or 16, wherein the disease is lung cancer
18. Use of a set of miRNAs according to any of the items 15 to 17 for diagnosing and/or prognosing of a disease in a subject.
19. Use of a set of miRNAs according to any of the items 15 to , wherein the disease is lung cancer
20. An isolated nucleic acid molecule selected from the group consisting of
   (a) a nucleotide sequence shown in SEQ ID NO: 1-222
   (b) a nucleotide sequence which is the complement of (a),
   (c) a nucleotide sequence which is the DNA complement of (a) or (b), and
   (d) a nucleotide sequence which has a sequence identity of at least 90% to (a) or (b) or (c)
21. An isolated nucleic acid molecule selected from the group consisting of
   (a) a nucleotide sequence shown in SEQ ID NO: 10-14, 16, 18-22, 24, 26-27. 29-30, 32-33, 35, 37-40, 43-47, 49, 51-52, 54, 56, 58, 60-61, 63-64, 66-67, 70-71, 74, 76, 79-80, 83, 85, 88-90, 94, 96-97, 99-100, 103-105, 108, 110, 114-115, 117, 120, 123, 127-128, 131-132, 134, 136, 139 142, 147, 151, 153, 155, 158, 162, 169, 171, 174-175, 177, 179, 181, 191, 197, 199, 205, 208, 212, 214, 218, 223
   (b) a nucleotide sequence of 16-32 nucleotides which is a fragment of the nucleotide sequence of (a),
   (c) a nucleotide sequence which is the complement of (a) or (b)
   (d) a nucleotide sequence which is the DNA complement of (a) or (b) or (c), and
   (e) a nucleotide sequence which has a sequence identity of at least 90% to (a) or (b) or (c) or (d)

### BRIEF DESCRIPTION OF THE DRAWINGS

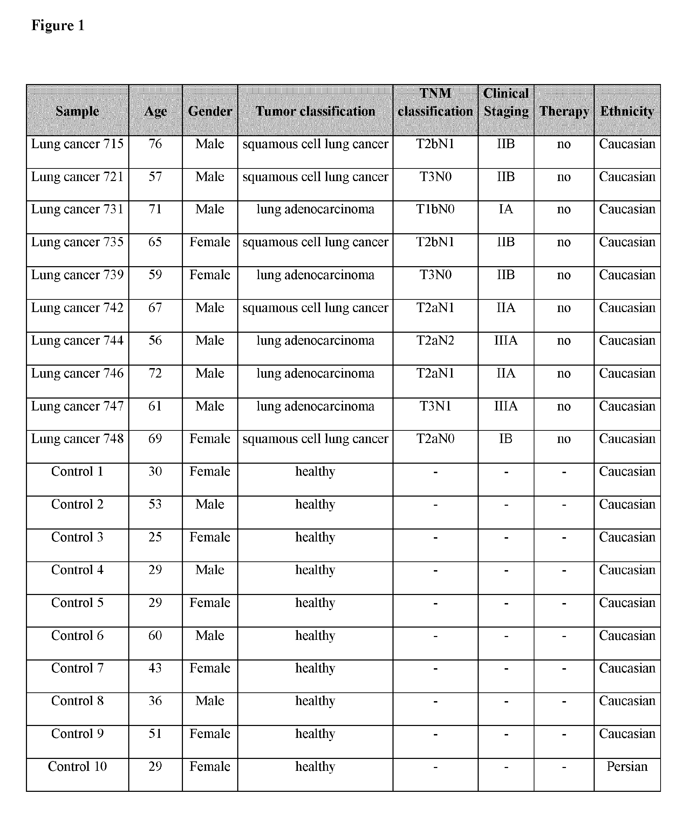

Figure 1: Characteristics of blood donors (healthy controls and lung cancer patients)

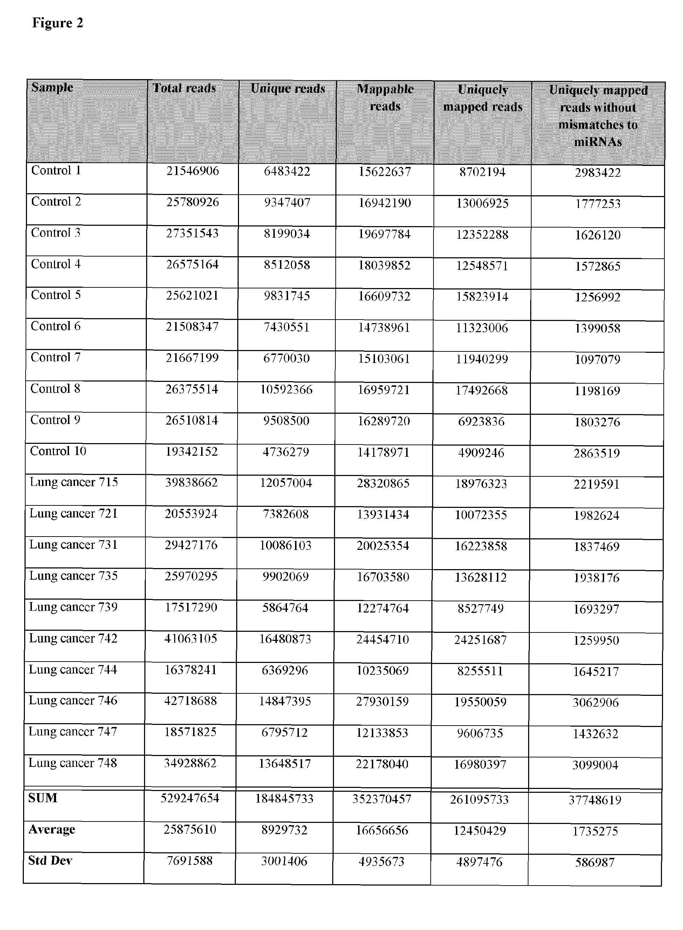

Figure 2: Sequencing reads obtained on the SOLID next-generation sequencing instrument when analyzing the small RNA fraction of the blood samples of healthy control subjects and lung cancer patients.
Figure 3: Differentially expressed microRNAs resulting from next-generation sequencing of healthy control and lung cancer patient blood samples (Fold Change = fold change expression between healthy control and lung cancer patient obtained from next-generation sequencing n SOLID; WMW_raw pval= p-value obtained when applying wmw-test (Wilcoxon Mann Whitney test), wmw_adj pval= adjusted p-value in order to reduce false discovery rate by Benjamini-Hochberg adjustment; AUC= Area under the curve; Fold Change microarray= fold change expression between healthy control and lung cancer patient obtained on geniom microarrays; concordance = concordance between results obtained with next-generation sequencing and microarray analysis)
Figure 4: Individual sequencing results (reads) obtained on the SOLID next-generation sequencing instrument when analyzing the small RNA fraction of the blood samples of healthy control subjects and lung cancer patients. Depicted are mayor (miRNA) or minor miRNAs (miRNA*) or miRNA precursor sequences that were previously unknown and were newly identified by next generation sequencing from blood samples of healthy control subjects and lung cancer patients (SEQ ID NO: = sequence identification number, miRNA: identifier of the miRNA; reads controls = sum of unique reads for healthy control subjects; reads Lung Cancer = sum of unique reads for lung cancer subjects; C1-C10 = unique reads for individual healthy control subjects C1-C10; C1-C10 = unique reads for individual lung cancer subjects LC 715, LC 748, LC 742, LC 721, LC 735, LC 746, LC 739, LC 747, LC 731, LC 744)

### EXAMPLES

The Examples are designed in order to further illustrate the present invention and serve a better understanding. They are not to be construed as limiting the scope of the invention in any way.

### Study population

For the next-generation sequencing approach, we obtained whole blood samples from ten patients with NSCLC and ten healthy individuals (Figure 1). Both cohorts showed a non-significant difference in gender distribution (Fishers Exact test p-value of 0.36).

For qRT-PCR we obtained lung cancer tissue from 16 different patients. We combined the RNA from those tissues to four pools, i.e., one squamous cell lung cancer pool, one adenocarcinoma pool, one large cell lung cancer pool, and one small cell lung cancer pool.

Considering the ethnic groups, all individuals were Caucasians with except of one Persian among the healthy blood donors.. The enclosed lung cancer patients did not undergo any radio-or chemotherapy before blood drawing and tumor resection, all tumor patients were smokers or former smokers with 7 to 80 packyears.

Local ethics committee has approved the analysis of blood and tissue from patients and controls and participants have given their informed consent. We collected 2.5-5 ml whole blood in TM PAXgene Blood RNA tubes (PreAnalytiX) and stored the samples at -20°C until extraction of total RNA. Further, we analyzed RNA isolated from 16 lung cancer tissue samples.

### Isolation of total RNA from blood cells and tissue

Blood of patients has been extracted as previously described [1,2] . In brief, 2.5 to 5 ml blood was extracted in PAXgene Blood RNA tubes (BD, Franklin Lakes, New Jersey USA) and centrifuged at 5000 x g for 10 min at room temperature. The miRNeasy kit (Qiagen GmbH, Hilden) was used to isolate total RNA including miRNA from the resuspended pellet according to manufacturer's instructions. The eluted RNA was stored at -70°C.

For the isolation of RNA from tissue, samples were homogenized in 2 ml QIAzoI lysis reagent and incubated for 5 min at RT. Then 200 µl chloroform were added, vortexed for 15 sec, and incubated for 2-3 min at RT. Subsequently, we followed the same protocol as applied for blood.

### Library Preparation

1.5 µg of total RNA was enriched for the fraction of small RNAs (10-40 nt) using Ambion's flashPAGE Fractionator, followed by sodium acetate precipitation. SOLiD internal adapters were ligated using 100 ng enriched fraction. After ligation, smallRNAs were transcribed into cDNA with Reverse Transcriptase. cDNA fragments between 60 and 80 nt (small RNAs + adaptors) were isolated from a 10% TBE Urea Gel (Novex-System, Invitrogen). RNA from gel slices was amplified with 15 PCR cycles using the same 5'-Primer for each sample and ten different 3'-Primers including the barcode sequences (SOLiD Multiplexing Barcoding Kit 01-16). A total of ten purified and barcoded DNA libraries was analyzed with a HS-DNA Chip in the Agilent Bioanalyzer 2100 and subsequently pooled in equimolar amounts.

### Next Generation Sequencing

The pooled libraries were diluted to a concentration of 41 pg/µl. DNA was amplified monoclonally on magnetic beads in an emulsionPCR. Emulsions were broken with butanol and the remaining oil was washed off the templated double-stranded beads. DNA on the bead surface was denatured to allow hybridization of the enrichment beads to the single stranded DNA. Using a glycerol cushion the null beads can be separated from the templated beads. After centrifugation, the enriched magnetic beads were in the supernatant. The enrichment-beads were separated from the magnetic beads by denaturation. The 3'-end was enzymatically modified for deposition on the sequencing slide. 700 Million Beads were loaded onto a Full Slide and sequenced on a SOLiD 4 analyzer.

### Mapping of reads

Mapping of SOLiD sequencing reads against known miRNAs and the genome was done using the RNA2MAP tool (version 0.5) from Applied Biosystems (http://solidsoftwaretools.com/gf/project/rna2map/). To use the default parameters of this mapping pipeline, we first trimmed the reads to a size of 35 nt. To reduce the overhead of computation, we reduced the amount of reads per sample to those being unique in the sample. The RNA2MAP pipeline included three steps: 1) reads are filtered against tRNAs, rRNAs, and other repetitive elements; 2) the remaining reads are mapped against the predicted precursor sequences of miRNAs from miRBase (version 16 [7-9]); 3) the remaining reads are mapped against the human genome (hg19). The mapped genome reads served as input for the prediction of novel miRNAs with miRDeep [10]. The predicted novel miRNA precursor sequences were added to the precursor sequences of miRBase and step 2 of the RNA2MAP pipeline was repeated to retrieve the counts for both the known and novel predicted precursor sequences.

### Prediction of novel miRNAs

For the prediction of novel miRNAs, we used a probabilistic model of miRNA biogenesis in combination with the frequency of RNA reads along the secondary structure of the miRNA precursor as implemented in miRDeep [10]. Previously, we transformed the output of the alignments of RNA2MAP to the so-called 'blastparsed' format of miRDeep. To this end, we removed the sequencing adaptor, converted the colorspace mapping into bases, re-counted the mismatches, adjusted the alignment length, and computed a bitscore and an E-value as described previously [11]. The miRDeep pipeline itself was run with default parameters using Randfold (v 2.0, [12]) and a fasta file containing the mature miRNA sequences from miRBase v16 (without human sequences) to improve accuracy and sensitivity. To reduce the number of false positive predictions, we ran 100 permutation tests and excluded a predicted novel miRNA if found in any of the permutation runs. The remaining putative novel miRNAs (p-value < 0.01) were mapped with BLAST (v 2.2.24, [13]) against known ncRNA and miRNA sequences from diverse sources (miRBase v16, snoRNA-LBME-db [14], ncRNAs from Ensembl "Homo_sapiens. GRCh37.59.ncrna.fa" ((ftp://ftp.ensembl.org/pub/release-59/fasta/homo_sapiens/ncrna/). NONCODE v2.0, [15]). We excluded sequences that aligned with more than 90% of their length (allowing 1 mismatch) to any of the ncRNA sequences.

### Distribution of miRNA reads across the miRNA precursors

Since we performed a size selection we do not intent to measure the expression level of the miRNA-precursor but of the mature miRNAs. The mapping of mature miRNA reads to the respective precursor sequence however offers the option to understand how the mature miRNA reads distribute along the precursor. To consider the distribution of reads mapping to a miRNA precursor, we computed for each precursor separately the coverage of each base position for lung cancer samples and controls. Likewise, we also computed for each base position of each precursor a significance value using Wilcoxon Mann-Whitney (WMW) test.

### Downstream analysis

To further evaluate the NGS miRNA profiles, we carried out statistical computations using R [16]. Shapiro-Wilk test has been applied to determine whether miRNA counts across all samples are normally distributed. To normalize samples standard quantile normalization has been applied to make the different sequencing runs comparable to each other. Expression of a miRNA i in a sample *j* has been measured as the normalized read count of this miRNAs in the respective sample. Grubbs test has been carried out for detecting outliers. Non-parametric WMW test has been performed for detecting differentially regulated miRNAs. To further assess the validity of the signature we have carried out non-parametric permutation tests. Here, the class labels have been randomly shuffled 100 times and the same analyses as for the original class labels have been carried out. A p-value was computed as the fraction of random runs with a likewise significant result as the original computations.

In addition to WMW analysis, we performed an analysis considering the total length of a miRNA precursor to identify possible novel miRNAs that derive from this precursor. In detail, we computed for each precursor *m* and each base i the WMW significance value for the respective position in the precursor at position i, testing the hypothesis that read counts of miRNA *m* at position i are significantly higher for lung cancer samples as compared for normal controls. For each miRNA precursor, we then counted the number of bases with WMW significance values < 0.05. Furthermore, the area under the receiver operator characteristics (AUC) curve has been computed for each miRNA. Cluster analysis has been done using the 'hclust' package.

For computing targets of deregulated miRNAs, the miRANDA algorithm has been applied and only miRNA-mRNA relations with p-values < 0.0001 have been considered [17]. To carry out gene set enrichment of target genes, we used GeneTrail and carried out a so-called over representation analysis [18, 19].

### REFERENCES

1. Meder, B., A. Keller, B. Vogel, J. Haas, F. Sedaghat-Hamedani, E. Kayvanpour, S. Just, A. Borries, J. Rudloff, P. Leidinger, E. Meese, H.A. Katus, and W. Rottbauer, MicroRNA signatures in total peripheral blood as novel biomarkers for acute myocardial infarction. Basic Res Cardiol, 2011. 106((1)): p. 13-23.
2. Leidinger, P., A. Keller, A. Borries, J. Reichrath, K. Rass, S.U. Jager, H.P. Lenhof, and E. Meese, High-throughput miRNA profiling of human melanoma blood samples. BMC Cancer. 10: p. 262.
3. Keller, A., P. Leidinger, J. Lange, A. Borries, H. Schroers, M. Scheffler, H.P. Lenhof, K. Ruprecht, and E. Meese, Multiple sclerosis: microRNA expression profiles accurately differentiate patients with relapsing-remitting disease from healthy controls. PLoS One, 2009. 4(10): p. e7440.
4. Keller, A., P. Leidinger, A. Borries, A. Wendschlag, F. Wucherpfennig, M. Scheffler, H. Huwer, H.P. Lenhof, and E. Meese, miRNAs in lung cancer - studying complex fingerprints in patient's blood cells by microarray experiments. BMC Cancer, 2009. 9: p. 353.
5. Hausler, S.F., A. Keller, P.A. Chandran, K. Ziegler, K. Zipp, S. Heuer, M. Krockenberger, J.B. Engel, A. Honig, M. Scheffler, J. Dietl, and J. Wischhusen, Whole blood-derived miRNA profiles as potential new tools for ovarian cancer screening. Br J Cancer. 103(5): p. 693-700.
6. Zhang, C., C. Wang, X. Chen, C. Yang, K. Li, J. Wang, J. Dai, Z. Hu, X. Zhou, L. Chen, Y. Zhang, Y. Li, H. Qiu, J. Xing, Z. Liang, B. Ren, K. Zen, and C.Y. Zhang, Expression Profile of MicroRNAs in Serum: A Fingerprint for Esophageal Squamous Cell Carcinoma. Clin Chem, 2010. 56((12)): p. 1871-9.
7. Griffiths-Jones, S., miRBase: the microRNA sequence database. Methods Mol Biol, 2006. 342: p. 129-38.
8. Griffiths-Jones, S., H.K. Saini, S. van Dongen, and A.J. Enright, miRBase: tools for microRNA genomics. Nucleic Acids Res, 2008. 36(Database issue): p. D154-8.
9. Griffiths-Jones, S., R.J. Grocock, S. van Dongen, A. Bateman, and A.J. Enright, miRBase: microRNA sequences, targets and gene nomenclature. Nucleic Acids Res, 2006. 34(Database issue): p. D140-4.
10. Friedlander, M.R., W. Chen, C. Adamidi, J. Maaskola, R. Einspanier, S. Knespel, and N. Raj ewsky, Discovering microRNAs from deep sequencing data using miRDeep. Nat Biotechnol, 2008. 26(4): p. 407-15.
11. Goff, L.A., J. Davila, M.R. Swerdel, J.C. Moore, R.I. Cohen, H. Wu, Y.E. Sun, and R.P. Hart, Ago2 immunoprecipitation identifies predicted microRNAs in human embryonic stem cells and neural precursors. PLoS One, 2009. 4(9): p. e7192.
12. Bonnet, E., J. Wuyts, P. Rouze, and Y. Van de Peer, Evidence that microRNA precursors, unlike other non-coding RNAs, have lower folding free energies than random sequences. Bioinformatics, 2004. 20(17): p. 2911-7.
13. Altschul, S.F., T.L. Madden, A.A. Schaffer, J. Zhang, Z. Zhang, W. Miller, and D.J. Lipman, Gapped BLAST and PSI-BLAST: a new generation of protein database search programs. Nucleic Acids Res, 1997. 25(17): p. 3389-402.
14. Lestrade, L. and M.J. Weber, snoRNA-LBME-db, a comprehensive database of human H/ACA and C/D box snoRNAs. Nucleic Acids Res, 2006. 34(Database issue): p. D158-62.
15. Liu, C., B. Bai, G. Skogerbo, L. Cai, W. Deng, Y. Zhang, D. Bu, Y. Zhao, and R. Chen, NONCODE: an integrated knowledge database of non-coding RNAs. Nucleic Acids Res, 2005. 33(Database issue): p. D112-5.
16. Team, R., R: A Language and Environment for Statistical Computing. 2008, R Foundation for Statistical Computing: Vienna.
17. Enright, A.J., B. John, U. Gaul, T. Tuschl, C. Sander, and D. S. Marks, MicroRNA targets in Drosophila. Genome Biol, 2003. 5(1): p. R1.
18. Keller, A., C. Backes, M. Al-Awadhi, A. Gerasch, J. Kuntzer, O. Kohlbacher, M. Kaufmann, and H.P. Lenhof, GeneTrailExpress: a web-based pipeline for the statistical evaluation of microarray experiments. BMC Bioinformatics, 2008. 9: p. 552.
19. Backes, C., A. Keller, J. Kuentzer, B. Kneissl, N. Comtesse, Y.A. Elnakady, R. Muller, E. Meese, and H.P. Lenhof, Gene Trail--advanced gene set enrichment analysis. Nucleic Acids Res, 2007. 35(Web Server issue): p. W186-92.

## Claims

1. A method for diagnosing and/or prognosing of a disease comprising the steps of:
(i) determining an expression profile of a set comprising at least two miRNAs representative for the disease in a blood sample from a subject, and
(ii) comparing said expression profile to a reference, wherein the comparison of said expression profile to said reference allows for the diagnosis and/or prognosis of the disease,
wherein at least one nucleotide sequences of the miRNAs comprised in the set is selected from the group consisting of SEQ ID NO: 1 to 222, a fragment thereof, and a sequence having at least 90% sequence identity thereto.

2. The method of claim 1, wherein the blood sample is a whole blood sample containing at least red blood cells, platelets and granulocytes

3. The method of any of the claims 1 or 2, wherein the disease is lung cancer

4. The method of claim 3, wherein further nucleotide sequences of the miRNAs comprised in the set are selected from the group consisting of SEQ ID NO: 223 to 254, a fragment thereof, and a sequence having at least 90% sequence identity thereto.

5. A set comprising polynucleotides for detecting a set comprising at least two miRNAs for diagnosing and/or prognosing of a disease in a blood sample from a subject, wherein at least one nucleotide sequences of the miRNAs comprised in the set is selected from the group consisting of SEQ ID NO: 1 to 222.

6. The set comprising polynucleotides of claim 5, wherein the blood sample is a whole blood sample containing at least red blood cells, platelets and granulocytes

7. The set comprising polynucleotides of any of the claims 5 or 6, wherein the disease is lung cancer

8. The set comprising polynucleotides of claim 7, wherein further nucleotide sequences of the miRNAs comprised in the set are selected from the group consisting of SEQ ID NO: 223 to 254

9. The set comprising polynucleotides according to any of the claims 5 to 8, wherein
(i) the polynucleotides comprised in the set are complementary to the miRNAs comprised in the set according to claims 5 or 8,
(ii) the polynucleotides comprised in the set are fragments of the polynucleotides comprised in the set according to (i), or
(iii) the polynucleotides comprised in the set have at least 90% sequence identity to the polynucleotide sequences of the polynucleotides comprised in the set according to (i) or polynucleotide fragments comprised in the set according to (ii).

10. Means for diagnosing and/or prognosing of a disease in a blood sample of a subject comprising :
(i) A set comprising polynucleotides for detecting a set comprising at least two miRNAs according to any of the claims 5 to 9 and
(ii) a biochip, a RT-PCT system, a PCR-system, a flow cytometer, Luminex system or a next generation sequencing system.

11. A kit for diagnosing and/or prognosing of a disease comprising
(i) means for determining an expression profile of a set comprising at least two miRNAs representative for the disease in a blood sample from a subject according to claim 10, and
(ii) at least one reference.

12. A set of at least 2 miRNAs isolated from a blood sample from a subject for diagnosing and/or prognosing of a disease, wherein at least one miRNAs is selected from the group consisting of SEQ ID NO: 1 to 222

13. The set of miRNAs of claim 12, wherein the blood sample is a whole blood sample containing at least red blood cells, platelets and granulocytes

14. The set of miRNAs of any of the claims 12 or 13, wherein the disease is lung cancer

15. Use of a set of miRNAs according to any of the claims 12 to 14 for diagnosing and/or prognosing of a disease in a subject.
